(19) **Europäisches Patentamt European Patent Office Office européen des brevets**

(11) **EP 3 881 814 B1**

(12) **EUROPEAN PATENT SPECIFICATION**

(45) Date of publication and mention of the grant of the patent:
**06.05.2026 Bulletin 2026/19**

(21) Application number: **20163636.2**

(22) Date of filing: **17.03.2020**

(51) International Patent Classification (IPC):
*A61F 13/532* (2006.01)    *A61F 13/534* (2006.01)
*A61F 13/53* (2006.01)

(52) Cooperative Patent Classification (CPC):
**A61F 13/5323; A61F 13/534;** A61F 2013/530532;
A61F 2013/530547; A61F 2013/530591;
A61F 2013/5349

(54) **ABSORBENT CORE COMPRISING A HIGH LOFT CENTRAL LAYER AND SUPERABSORBENT PARTICLES**

ABSORBIERENDER KERN MIT EINER ZENTRALEN SCHICHT MIT HOHEM VOLUMEN UND SUPERABSORBIERENDEN TEILCHEN

NOYAU ABSORBANT COMPRENANT UNE COUCHE CENTRALE À EFFET GONFLANT ÉLEVÉ ET DES PARTICULES SUPERABSORBANTES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**22.09.2021 Bulletin 2021/38**

(73) Proprietor: **The Procter & Gamble Company Cincinnati, OH 45202 (US)**

(72) Inventors:
• **Kamphus, Juliane**
  **65824 Schwalbach am Taunus (DE)**
• **Peri, Andrea**
  **65824 Schwalbach am Taunus (DE)**
• **Seeboth, Simone**
  **65824 Schwalbach am Taunus (DE)**

(74) Representative: **P&G Patent Germany Procter & Gamble Service GmbH Sulzbacher Straße 40 65824 Schwalbach am Taunus (DE)**

(56) References cited:
**EP-A1- 2 901 992      EP-B1- 2 679 208**
**US-A1- 2015 080 821      US-A1- 2016 175 169**

• **ANONYMOUS: "Schauch HVDE 235 Superabsorber", 25 December 2014 (2014-12-25), XP055724736, Retrieved from the Internet <URL:https://www.amazon.de/ Schauch-Superabsorber-Feuchtigkeitssch�t zer-Wasserspeicherung-Betreuungsbereichen/ dp/B00VD0N378> [retrieved on 20200824]**

**Description**

FIELD OF THE INVENTION

The invention relates to absorbent cores and their use in personal hygiene absorbent articles. The absorbent cores may be in particular used in baby diapers.

BACKGROUND OF THE INVENTION

Absorbent articles for personal hygiene such as disposable baby diapers, training pants for toddlers or adult incontinence undergarments, are designed to absorb and contain body exudates, in particular urine. These absorbent articles comprise several layers providing different functions, typically including a topsheet, a backsheet and in-between an absorbent core, among other layers.

The absorbent core should be able to absorb and retain the exudates for a prolonged amount of time, for example overnight for a diaper, minimize re-wet to keep the wearer dry, and avoid soiling of clothes or bed sheets. Absorbent cores have typically comprised a blend of comminuted wood pulp cellulose fibers with superabsorbent polymers (SAP) particles, also called absorbent gelling materials (AGM), as absorbent material.

Absorbent cores without fluff cellulose fibers (also called "airfelt-free" cores) have been more recently proposed. The SAP particles may be for example enclosed within discrete pockets formed between two substrates (see e.g. WO95/11654, Tanzer et al.). It has also been proposed to immobilize SAP particles with a microfibrous adhesive network to a nonwoven substrate by an adhesive (see e.g. WO2008/155699A1, Hundorf et al. and US2015/0080821A1, Peri et al.). More recently, airfelt-free cores have been disclosed comprising a high loft central layer with SAP distributed therein (see e.g. WO2016/106,021A1, Bianchi et al.). These cores are typically made by distributing a layer of SAP particles on each side of a high loft nonwoven and laminating both sides with a tissue paper or a nonwoven to immobilize the particles (the process for example illustrated in Fig. 3 of WO2020/025401 (BASF, Ge et al.)). Other recent central layer core publications are WO2020/032280, WO2020/032281, WO2020/032282, WO2020/032283 and WO2020/032284 (Nippon SHOKUBAI). EP2,901,992A1 (Ontex) discloses multi-layer absorbent material.

There is a continuous need to improve the performances of absorbent cores, in particular in terms of absorption speed and capacity, wearer comfort, low rewet and flexibility, while keeping the overall costs of manufacture as low as possible.

SUMMARY OF THE INVENTION

The present invention is directed to an absorbent core extending in a transversal direction and a longitudinal direction, having a thickness in a vertical direction, and which comprises a liquid-permeable top layer, a bottom layer, and a central layer sandwiched between the top layer and the bottom layer. The central layer is a high loft layer, such as a carded nonwoven, having a density at a pressure of 4.14 kPa below 0.20 g/cm$^3$. The absorbent core comprises superabsorbent polymer particles (SAP), which are at least partially distributed within the central layer. The superabsorbent polymer particles contained in the core have a time to reach an uptake of 20 g/g (SAP T20) of less than 220s, as measured according to the SAP K(t) Test Method as described herein.

The absorbent core may also have a time to reach an uptake of 15 g/g (Core T15) of less than 200 s, as measured according to the Absorbent Core K(t) Test Method as described herein. The absorbent core may also have a permeability (Core K20) of more than 6.0 10$^{-8}$ cm$^2$, preferably more than 8.0 10$^{-8}$ cm$^2$, as measured according to Absorbent Core K(t) Test Method as described herein. The Core T15 and Core K20 may be measured directly on the core, whereas the SAP T20 is measured on the SAP separately.

The absorbent cores of the invention have fast speed of absorption, especially in the first and second gush of a typical fluid insult. This reduces the risk of early leakage, i.e. leakage at low loading. The absorbent cores of the invention also have a lower liquid distribution length relative to other absorbent cores, i.e. are less wetted at front and back, while maintaining acceptable Rewet performance at the loading point (approximately the center of the absorbent core). This is beneficial to keep the skin of the wearer dryer at the contact area of the front and back of the absorbent structure.

The absorbent core may comprise at least 60% by weight of SAP, in particular at least 70%, or even at least 80% of SAP, or even at least 90% of SAP, relative to the total weight of the core. The high loft central layer may be formed entirely from synthetic fibers, and may be substantially free of fluff cellulose fibers, although natural or natural-sourced fibers such a cellulose or cotton fibers or viscose fibers may also be present in the central layer and/or the top layer and/or the bottom layer.

The top layer and the bottom layer are typically a nonwoven or a tissue paper. Low basis weight tissue paper for example is readily available and a relatively cheap substrate. The absorbent core may also comprise a wrapping layer that completely covers the bottom layer or top layer and forms a C-wrap around the longitudinally extending side edges of the central layer to at least partially cover the top layer or bottom layer respectively and better immobilize the SAP particles within the absorbent core. A wrapping layer can provide an improved containment of the SAP thus preventing losses on the side edges of the core. Alternatively, such a C-wrap may also be formed by the top layer or the bottom layer.

The absorbent cores may also comprise a dual layer construction comprising a first central high loft layer and a second central high loft layer. This construction may provide additional benefit for example in terms of SAP immobilization and a higher quantity of SAP particles may be distributed within two layers. These and other optional features of the invention will be described in the following description.

BRIEF DESCRIPTION OF THE DRAWINGS

Fig. 1 shows a top view of an exemplary absorbent core with the top and central layers partially removed;
Fig. 2a-c shows different possible schematic cross-sectionals view of the absorbent core of Fig. 1 in exploded view ;
Fig. 3 shows a schematic cross-sectional view of the absorbent core of Fig. 2a and a core wrapping layer;
Fig. 4 shows a schematic cross-sectional of an alternative absorbent core comprising two central high loft layers;
Fig. 5 shows a schematic cross-sectional of an absorbent article comprising the absorbent core of Fig. 4 and a core wrapping layer;
Fig. 6a shows a micro-CT scan of a circular sample of an inventive core;
Fig. 6b shows a projection of the micro-CT scan in the xz plane;
Fig. 6c shows a projection of the micro-CT scan in the yz plane;
Fig. 7a shows a diagram showing the concentration of SAP particles in the z direction for an exemplary core;
Fig. 7b shows a diagram showing the concentration of SAP particles in the z direction for a comparative core;
Fig. 8 shows a micro-CT scan of the SAP particles in a top region of the central layer of the exemplary core;
Fig. 9 shows a micro-CT scan of the SAP particles in a middle region of the central layer of the of the core;
Fig. 10 shows a micro-CT scan of the SAP particles in a bottom region of the central layer of the of the core;
Fig. 11 is a partial cross-sectional side view of a suitable permeability measurement system for conducting the Urine Permeability Measurement Test;
Fig. 12 is a cross-sectional side view of a piston/cylinder assembly for use in conducting the Urine Permeability Measurement Test;

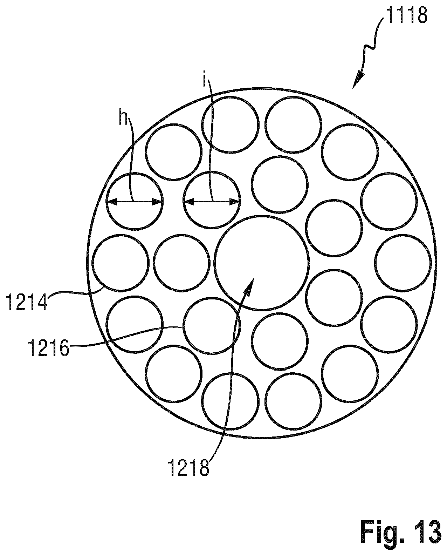

Fig. 13 is a top view of a piston head suitable for use in the piston/cylinder assembly shown in Fig. 12;
Fig. 14 is a cross-sectional side view of the piston/cylinder assembly of Fig. 12 placed on fritted disc for the swelling phase;
Fig. 15 is a partial cross-sectional side view of a suitable permeability measurement system for conducting the Dynamic Effective Permeability and Uptake Kinetics Measurement Test;
Fig. 16 is a cross-sectional side view of a piston/cylinder assembly for use in conducting the Dynamic Effective Permeability and Uptake Kinetics Measurement Test;
Fig. 17 is a top view of a piston head suitable for use in the piston/cylinder assembly shown in Fig. 15;
Fig. 18 is a schematic sketch of a process for making an absorbent core of the invention.

DETAILED DESCRIPTION OF THE INVENTION

**Introduction**

As used herein, the terms "comprise(s)" and "comprising" are open-ended; each specifies the presence of the feature that follows, e.g. a component, but does not preclude the presence of other features, e.g. elements, steps, components known in the art or disclosed herein. These terms based on the verb "comprise" should be read as encompassing the narrower terms "consisting essentially of" which excludes any element, step or ingredient not mentioned which materially affect the way the feature performs its function, and the term "consisting of" which excludes any element, step, or ingredient not specified. Any preferred or exemplary embodiments described below are not limiting the scope of the claims, unless specifically indicated to do so. The words "typically", "normally", "preferably", "advantageously", "in particular" and the likes also qualify features which are not intended to limit the scope of the claims unless specifically indicated to do so.

As used herein, the terms "nonwoven", nonwoven layer" or "nonwoven web" are used interchangeably to mean an engineered fibrous assembly, primarily planar, which has been given a designed level of structural integrity by physical and/or chemical means, excluding weaving, knitting or papermaking (ISO 9092:2019 definition). The directionally or randomly orientated fibers, are bonded by friction, and/or cohesion and/or adhesion. The fibers may be of natural or synthetic origin and may be staple or continuous filaments or be formed in situ. Commercially available fibers have diameters ranging from less than about 0.001 mm to more than about 0.2 mm and they come in several different forms such as short fibers (known as staple, or chopped), continuous single fibers (filaments or monofilaments), untwisted bundles of continuous filaments (tow), and twisted bundles of continuous filaments (yam). Nonwoven webs can be formed by many processes such as meltblowing, spunbonding, solvent spinning, electrospinning, carding and airlaying. The basis weight of nonwoven webs is usually expressed in grams per square meter ($g/m^2$ or gsm).

## General description of the absorbent core

As used herein, the term "absorbent core" refers to an individual component comprising an absorbent material to absorb and retain body fluid, in particular urine. The absorbent core typically has the most absorbent capacity of the components of the absorbent article and comprises all, or at least the majority of, superabsorbent polymer (herein referred to as "SAP") particles. The terms "absorbent core" and "core" are herein used interchangeably. Some absorbent products may comprise two or more distinct absorbent cores but typically there is only one absorbent core in an absorbent product such as a diaper.

The absorbent cores of the invention are substantially planar. By substantially planar, it is meant that the absorbent core can be laid flat on a planar surface and primarily extend in an x and an y direction. The absorbent cores may also be typically thin and conformable, so that they can also be laid on a curved surface for example a drum during the making process, or stored and handled as a continuous roll of stock material comprising a plurality of cores before being converted into an absorbent article.

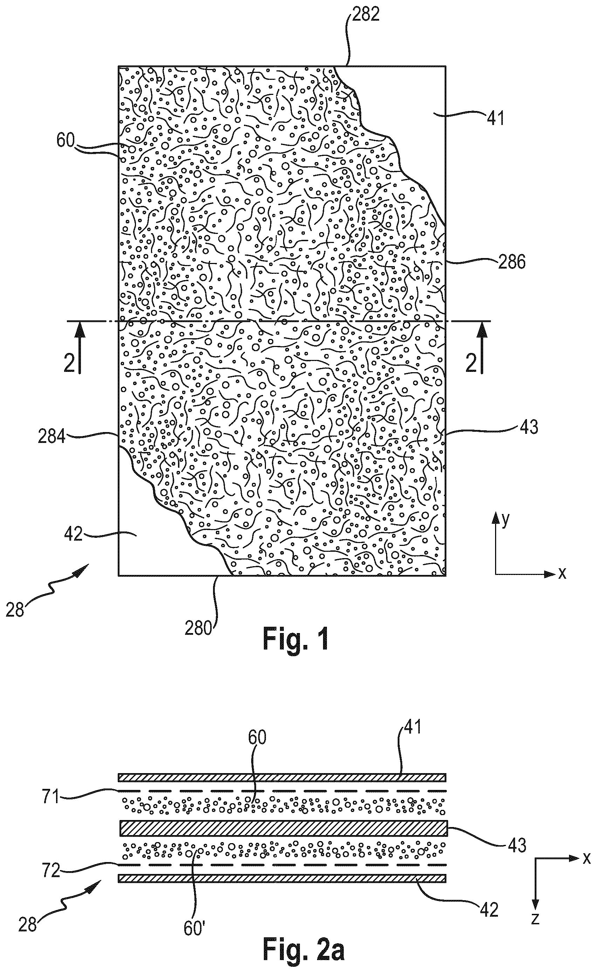

For ease of discussion, the exemplarily absorbent core of Fig. 1 is represented in a flat state. The absorbent core's height in the z direction is small relative to its other dimensions in the transversal direction x and the longitudinal direction y. Unless otherwise indicated, dimensions and areas disclosed herein apply to the core in this flat-out configuration.

For ease of discussion, the absorbent cores, articles and processes of the invention will be discussed with reference to the Figures and the numerals referred to in these Figures; however these are not intended to limit the scope of the claims unless specifically indicated.

## High loft central layer 43

The absorbent cores of the invention comprise a high loft central layer 43, as first illustrated in Figs. 1-2. The term "high loft" refers to low density bulky fabrics, as compared to flat, paper-like fabrics. High loft webs are characterized by a relatively high porosity. This means that there is a relatively high amount of void space between the fibers in which superabsorbent polymer particles can be distributed. The high loft layer (without the superabsorbent particles) of the invention has a density at a pressure of 4.14 kPa (0.6 psi) below 0.20 $g/cm^3$, in particular ranging from 0.01 $g/cm^3$ to 0.20 $g/cm^3$, or from 0.05 $g/cm^3$ to 0.15 $g/cm^3$, or from 0.0.10 $g/cm^3$ to 0.14 $g/cm^3$. The high loft layer (without the superabsorbent particles) of the invention may have a density at a pressure of 2.07 kPa (0.3 psi) below 0.20 $g/cm^3$, in particular ranging from 0.05 $g/cm^3$ to 0.15 $g/cm^3$, or from 0.08 $g/cm^3$ to 0.13 $g/cm^3$. The high loft layer (without the superabsorbent particles) of the invention may have a density at a pressure of 0.83 kPa (0.12 psi) below 0.15 $g/cm^3$, in particular ranging from 0.01 $g/cm^3$ to 0.15 $g/cm^3$, or from 0.05 $g/cm^3$ to 0.12 $g/cm^3$ or from 0.08 $g/cm^3$ to 0.10 $g/cm^3$. The density can be calculated by dividing the basis weight of the high loft layer by its thickness measured at the respective pressure as indicated (see the method details further below in the "test procedure" section).

The central layer is preferably a nonwoven, but other types of high loft material are not excluded. The central layer may comprise or consist of synthetic fibers, optionally mixed with natural fibers such as cellulose or cotton fibers or viscose fibers for example. The central layer may be substantially free of free cellulose fibers which are not integrated with the other fibers of the nonwoven. The amount of such free cellulose fibers in the absorbent core may be less than 10 percent by weight of the total absorbent core, or less than 5 percent by weight of the total absorbent core, or less than 1 percent by weight of the total absorbent core, or completely free of such free cellulose fibers. The high loft material may comprise at least 10%, 30% 50%, 70%, 90% and up to 100% by weight of the high loft layer, of synthetic fibers.

The fibers forming the central layer may be made partially or entirely of a relatively resilient synthetic fibers, in particular polypropylene (PP), polyamide (PA, such as nylons) or polyethylene terephthalate (PET) fibers. The diameter of the fibers may for example range from 0.01 mm to 0.50 mm.

The thickness, basis weight and density of the central layer 43 are typically homogenous in both transversal direction (x) and longitudinal direction (y). The orientation of fibers in the central layer 43 may be in-homogenous such as predominant orientation of fibers into one direction x or y such as in carded nonwovens. Furthermore, the fiber orientation in the central layer 43 in thickness direction z may be different versus the predominant orientation in one or both directions x and/or y. The high loft layer may in particular have a thickness of at least 0.30 mm, in particular ranging from 0.30 mm to 2.00 mm, or from 0.50 mm to 1.5 mm, as measured at a pressure of 4.14 kPa (0.6 psi) (according to the test method described further below). The high loft layer may in particular have a thickness ranging from 0.30 mm to 2.50 mm or from 0.5 to 2.0 mm or from 0.7 to 1.3 mm, as measured at a pressure of 0.83 kPa (0.12 psi) (according to the test method described further below). The basis weight of the high loft central layer may for example range from 15 gsm to 500 gsm, in particular from 30 gsm to 200 gsm, for example 50 gsm to 120 gsm. The values indicated herein for the central layer are considered for the high loft material taken in isolation, that is before the SAP particles have been deposited between the fibers or an adhesive applied to it. When the absorbent core comprises two or more high loft central layers, these may be the same or different.

While the invention is not limited to a specific type of nonwoven or fibers, a particular example of suitable nonwoven layer are through-air bonded carded webs ("TABCW'). "Bonded carded web" refers to nonwovens that are made from staple fibers that are sent through a combing or carding unit, which separates and generally aligns the staple fibers in the machine direction to form a generally machine direction-oriented fibrous nonwoven web. This web is then drawn through a heated drum, creating bonds throughout the fabric without applying specific pressure (through air bonding process). The TABCW material provides a low density, lofty through-air bonded carded web.

The TABCW material can for example comprise about 3 to about 10 denier staple fibers. Examples of such TABCW are disclosed in WO2000/71067 (KIM DOO-HONG et al.). TABCW are also available directly from all usual suppliers of nonwoven webs for use in absorbent articles, for example Fitesa Ltd or Fiberweb Technical Nonwovens. In a carded nonwoven, the fibers in the web are aligned predominantly in the machine direction and have a more uniform fiber alignment than other nonwovens, which results in greater stability and internal bond strength especially in machine direction. The bonding technique chosen influences the integrity of the fabric. Through-air bonded carded web have excellent softness, bulk and compressibility, and rapid strike through and good rewet. Synthetic, natural and recycled fibers in a wide range of deniers can be used. Soft PE/PP bicomponent staple fibers may in particular be used.

The high loft layer may also be a spunmelt nonwoven. Spunmelt is a generic term describing the manufacturing of nonwoven webs directly from thermoplastic polymers. It encompasses two processes and the combination of both: spunlaid (also known as spunbond) nonwoven and meltblown nonwoven. In a spunlaid process, polymer granules are melted and molten polymer is extruded through spinnerets. The continuous filaments are cooled and deposited onto a conveyor to form a uniform web. Some remaining temperature can cause filaments to adhere to one another, but this cannot be regarded as the principal method of bonding. The spunlaid process has the advantage of giving nonwovens greater strength, but raw material flexibility is more restricted. Co-extrusion of second components is used in several spunlaid processes, usually to provide extra properties or bonding capabilities. In meltblown web formation, low viscosity polymers are extruded into a high velocity airstream on leaving the spinneret. This scatters the melt, solidifies it and breaks it up into a fibrous web.

The central layer 43 has a front edge 280, a back edge 282 and two longitudinally-extending side edges 284, 286. The front and back edges are typically shorter than the side edges. The front edge of the central layer corresponds to the edge intended to be placed towards the front edge of the absorbent article in which the core is or will be integrated. The superabsorbent material may be distributed in higher amount towards the front half of the central layer relative to the back half of the central layer. This is because there is typically more fluid discharged towards the front of the article in which a core will be incorporated. In addition to the profiled SAP distribution in the longitudinal direction (y), the SAP may be also be profiled in the transversal direction (x). Of course, the SAP may be also homogenously distributed in the transversal (x) and the longitudinal direction (y), which simplifies production: in that case any of the two shorter sides may be considered as the front edge and the opposite side will be the back edge. The absorbent cores may comprise one, two or more such high loft central layer. An absorbent core comprising two high loft central layers is discussed further below.

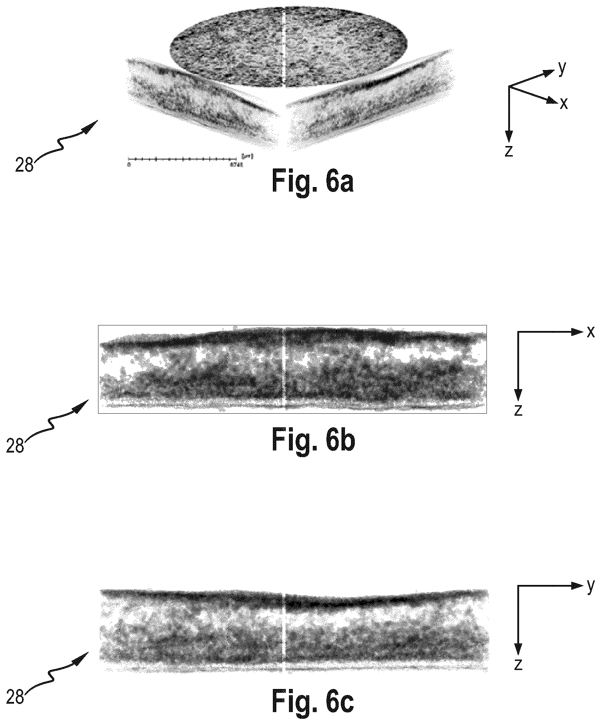

The central layer (or layers) serves as substrate for the SAP particles 60 which are at least partially distributed within its pores. The SAP particles may be substantially uniformly blended across the thickness of the high loft layer. However, the SAP particles may be distributed heterogeneously in the vertical direction. The SAP particles are typically deposited on one side of the nonwoven and drawn into the high loft nonwoven for example by gravity or a negative pressure on the opposite side of the nonwoven. In this way, some particles remain close to the surface of the high loft central layer and other, typically smaller, particles may penetrate deeper within the fiber network of the high loft nonwoven. The SAP particles which are not trapped within the pores of the high loft but remain at the surface may be further immobilized by a layer of adhesive 71 or 72. The adhesive is typically applied on the top and bottom layers first before being combined while still tacky with the high loft central layer. Typically the SAP particles are applied sequentially on the high loft layer from each side of the high loft layer as a first layer 60 of SAP and a second layer 60' of SAP. This process of particle deposition may result in a SAP z-distribution pattern inside the central layer comprising two or more peaks of density separated by at least one buffer zone, when seen in the z direction. Such a distribution is illustrated in the absorbent core of example of Figs. 6-10, which although has been obtained by deposition of the SAP on one side is believed to be representative of an absorbent core as shown in Fig. 2a-2c where two layers of the SAP have been sequentially distributed within the high loft layer.

**Top layer 41 and bottom layer 42**

The high loft central layer 43 is sandwiched between a top layer 41 and a bottom layer 42. The top layer 41 is on the side of the core intended to be placed closest to the wearer-facing side of the absorbent article. The top layer is thus liquid-permeable, so that a fluid can easily reach the central layer through the top layer during use. The bottom layer is positioned on the other side of the central layer. It may be liquid-permeable or liquid impermeable. The top layer and the bottom layer provide a cover on both sides of the central layer for preventing the SAP particles from falling out of the high loft during the core and article making process and/or during use of the absorbent article.

The top and bottom layers may be made of a relatively thin and cheap material, as are commonly used for the production of conventional cores. The top and bottom layers may be for example a tissue paper (airfelt or wetlaid) having a basis weight ranging for example from 5 to 100 gsm, in particular 10 to 40 gsm. The top and bottom layers may also be formed from a low basis weight nonwoven web having a basis weight of between 5 gsm and 30 gsm, such as a carded nonwoven, spunbond nonwoven ("S") or meltblown nonwoven ("M"), and laminates of any of these. For example, spunmelt polypropylene nonwovens are suitable, in particular those having a laminate web SMS, or SMMS, or SSMMS, structure, and having a basis weight range of about 5 gsm to 20 gsm. Such materials are for example disclosed in US7,744,576, US2011/0,268,932A1, US2011/0319848A1 and US2011/0,250,413A1. Nonwovens materials are typically inherently hydrophobic, and the top layer may thus be treated to render it hydrophilic, for example by treating it with a surfactant or other methods as is known in the art. The top layer and the bottom layer may be made of the same or different material, optionally with the top layer or bottom layer treated differently to render to the top layer more hydrophilic than the bottom layer.

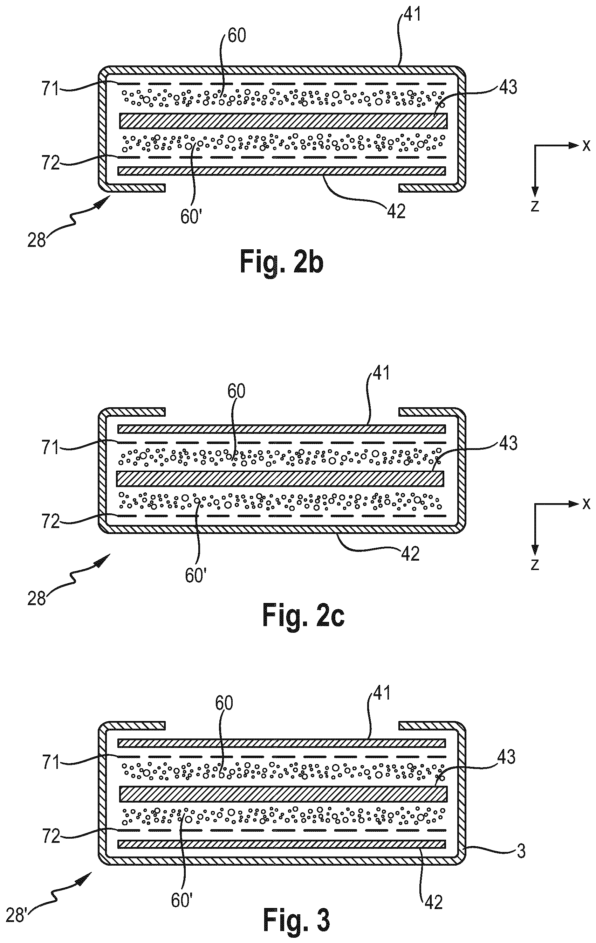

The top layer 41 can be wider than the bottom layer 42 so that this excess material can be folded around the longitudinal side edges 284, 286 of the core to form a C-wrap seal over the bottom layer 42, as illustrated in Fig. 2b. Alternatively the bottom layer 42 can be wider than the top layer 41 so that this excess material can be folded around the longitudinal side edges 284, 286 of the core to form a C-wrap seal over the top layer 41, as illustrated in Fig. 2c.

In addition to the top layer and the bottom layer, the absorbent core can further comprise a wrapping layer 3 forming a C-wrap around the longitudinally extending side edges 284, 286 of the core, as shown in Fig. 3. By "C-wrap", it is meant that the layer covers at least the top side or bottom side of the core, extends along its side edges to form flaps that are then folded and attached, typically by gluing, over the opposite side of the core. The wrapping layer 3 may thus have a cross-section similar to the letter C (when rotated 90°). A C-wrap construction may further help containing the SAP particles during the making or wearing of the absorbent article. The wrapping layer may for example be made of a low basis weight nonwoven layer, for example having a basis weight of from 5 to 40 gsm, in particular from 8 to 25 gsm, in particular a SMS nonwoven, but other materials are of course possible. The wrapping layer 3 has been represented in Fig. 3 as extending from the bottom side of the core and having flaps folded over the top side of the core. The inverted configuration is also possible, with the C-wrapped layer 3 extending from the top side and with the flaps folded over the bottom side. The folded flaps may end and be attached in the vicinity of the longitudinally extending side edges of the core or may be longer than represented to that they overlap and attached to another. It is also considered that a C-wrap construction may be formed by one of the top layer or bottom layer extending transversally along the longitudinally extending side edges of the core and forming flaps as described for the wrapping layer 3. The presence of a wrapping layer is optional but is preferred especially if the top layer and the bottom layer are not sealed along their longitudinal sides.

The top layer 41 and/or the bottom layer 42 may be attached to the central layer 43. A layer of glue 71 may be for example applied between the top layer and the central layer 43. Any type of conventional glue and glue application method may be used. Typically, a hot melt glue may be sprayed on substantially the whole of the surface of the layers before putting the two layers in close contact so that they become attached. The glue may also be applied by a contact method to one of the layers, in this case in particular the top or bottom layer, typically by slot-coating a series of parallel thin lines of glue in the machine direction (y direction). A layer of glue 72 may also be similarly applied between the bottom layer 42 and the central layer 43. These layers of glue also have the advantages that they can immobilize the SAP particles in the dry state that have not penetrated within the central layer during the making of the core.

**Superabsorbent material particles 60**

The central layer comprises a superabsorbent polymer in the form of particles 60 at least partially distributed within the fibers of the high loft layer. The term "superabsorbent polymer" (herein abbreviated as "SAP" in the singular and plural form) refers herein to absorbent materials that can absorb at least 10 times their weight of an aqueous 0.9% saline solution as measured using the Centrifuge Retention Capacity (CRC) test (EDANA method NWSP 241.0.R2 (19)). The SAP preferably have a CRC value of at least 15 g/g. The SAP of the invention may have a CRC which is less than 35 g/g, in particular less than 32 g/g.

SAP are typically water-insoluble but water-swellable cross-linked polymers capable of absorbing large quantities of fluids. SAP are in particulate form so as to be flowable in the dry state. Typical particulate SAP are polyacrylate polymers, however it is not excluded that other polymer materials may also be used. For example, starch-based particulate absorbent polymer material may also be used, as well polyacrylamide copolymer, ethylene maleic anhydride copolymer, cross-linked carboxymethylcellulose, polyvinyl alcohol copolymers, cross-linked polyethylene oxide, and starch grafted copolymer of polyacrylonitrile.

SAP may be polyacrylates and polyacrylic acid polymers that are internally and/ or surface cross-linked. The superabsorbent polymer of the invention may be selected from polyacrylates and polyacrylic acid polymers that are internally and surface cross-linked. The superabsorbent polymers can be internally cross-linked, i.e. the polymerization is carried out in the presence of compounds having two or more polymerizable groups which can be free-radically copolymerized into the polymer network. Exemplary superabsorbent polymer particles of the prior art are for example described in WO2006/083584, WO2007/047598, WO2007/046052, WO2009/155265, WO2009/155264. Preferably, the SAP particles comprise crosslinked polymers of polyacrylic acids or their salts or polyacrylates or derivatives thereof.

The SAP particles may be relatively small (under 1 mm in their longest dimension) in their dry state and may be roughly circular in shape, but granules, fibers, flakes, spheres, powders, platelets and other shapes and forms are also known to persons skilled in the art. Typically, the SAP may be in the form of spherical-like particles. The absorbent material may thus consist or consist essentially of the SAP distributed within the high loft nonwoven.

At least a portion of the SAP particles may be agglomerated, as e.g. taught in EP3,391,961A1 (Kamphus, P&G). The agglomerated superabsorbent polymer particles may be obtained by various methods. Agglomerated particles may be for example obtained by aggregating the precursor particles with an interparticle crosslinking agent reacted with the polymer material of the precursor particles to form crosslink bonds between the precursor particles have been for example disclosed in US5,300,565, US5,180,622, (both to Berg), US5,149,334, US5,102,597 (both to Roe), US5,492,962 (Lahrman). Other ways to obtain agglomerated SAP particles are for example described in EP3056521B1 (Kim et al.), EP1512712B1 (Koji et al.), US10414876B2 (Jang et al.), US7429009B2 (Nagasawa et al), EP220224911 (Higashimoto et al), EP2011803B1 (Handa et al).

Agglomerated superabsorbent polymer particles may also be obtained by a method comprising the steps of providing superabsorbent polymer particles and mixing the superabsorbent polymer particles with a solution comprising water and a multivalent salt having a valence of three or higher. This method is further disclosed EP2,944,376A1.

. The superabsorbent polymer particles of the core of the invention may in particular comprise at least 5%, or at least 10%, or at least 20%, or at least 30%, or at least 40%, or at least 50% by weight of the agglomerated superabsorbent polymer particles

Suitable precursor superabsorbent polymer particles may for example be obtained from inverse phase suspension polymerizations as described in US4,340,706 and US5,849,816 or from spray- or other gas-phase dispersion polymerizations as described in US2009/0192035, US2009/0258994 and US2010/0068520. In some embodiments, suitable precursor superabsorbent polymer particles may be obtained by production processes as is more particularly described from page 12, line 23 to page 20, line 27 of WO 2006/083584.

The surface of the SAP particles may be coated. The surface of the SAP may be surface crosslinked. The SAP particles may also comprise surface and/or edge modified clay platelets. Preferably, the clay platelets are montmorillonite, hectorite, laponite or mixtures thereof. Preferably, the clay platelets are laponite. The SAP may comprise from 0.1 to 5% by weight of clay platelets with modified surfaces and/or edges compared to the weight of the precursor superabsorbent polymer particles.

As claimed in the present invention, the SAP used in the core have a time to reach an uptake of 20 g/g (SAP T20) of less than 220 s as measured by the SAP K(t) test method described below. The SAP may in particular have a SAP T20 of from 100 s to 220 s. The SAP T20 values may be less than 200 s, or less than 180 s, or less than 160 s. The time T20 may also be of at least of 100 s, 104 s, 120 s or 140 s, and any combinations of these upper and lower values to form a range, e.g. of from 100 s to 200 s.

SAP having the required SAP T20 can be synthesized using for example the teaching of WO2015/041,784A1 which discloses SAP having a T20 ranging of from 104 s to 211 s. The SAP having the required SAP T20 may also be acquired directly from conventional SAP suppliers. For example, the inventive example below uses a SAP bought via Amazon under product name SCHAUCH HVDE 235, "Der Alleskoenner", having a measured SAP T20 of 165 s.

Unless otherwise indicated, the values indicated herein to qualify the SAP (e.g. SAP T20, CRC, AAP, ...) refer to the properties of the whole of the SAP used within the absorbent core. For example, if a first layer 60 and a second layer of SAP 60' is used to make the core, and the SAP used are different in each layer, the values to qualify the SAP of the core is based on the average value for these first and second SAP. In practice, a blend of the different SAP in the proportion used in the core is used to make the measurement.

High loft absorbent cores have an open pore structure and it has been suggested that SAP having a lower permeability may thus be advantageously used to take benefits of this property (see e.g. WO2016/106,021A1). This is however not without drawback. The inventors have found that liquid can quickly spread from the point of insult towards the front and back of the absorbent core during usage. Hence, the urine may escape the absorbent core through the front and back ends, leading to leakage, or at least create higher zones of rewet at the front and back of the absorbent core, leading to insufficient dryness and lack of comfort for the wearer. The inventors have now found that this drawback of undesired liquid spreading can be successfully addressed when using SAP having a relatively low SAP T20 value, as illustrated in the examples below. While not wishing to be bound by theory, the inventors believe that the low SAP T20 value are characteristics of SAP that can quickly absorb fluid even when the SAP particles are in close contact and/or under pressure and this prevents the fluid from spreading as far in the longitudinal direction of the absorbent core as with other type of SAP. While not wishing to be bound by theory, the inventors believe that the contact of SAP particles to the fibers of the central layer limit the speed of absorption of the SAP particles as fluid cannot freely penetrate into the particle at the contact area and the fiber network of the central layer creates swelling constraints onto the swelling SAP particles. The inventors believe that SAP with low SAP T20 values have the ability to overcome this negative effect of the fiber network of the central layer. The inventors further believe that other properties such as AAP@0.3 psi which are typically used to express the ability of SAP particles to work against moderate pressure are not suitable to achieve the benefit of the invention.

The SAP K(t) Test Method is also useful to determine other SAP parameters, which may also be advantageously used in the present invention. The uptake of the SAP at 20 min (U20) may be in particular of at least 22 g/g, or at least 24 g/g, or at least 28 g/g or at least 30 g/g, or of from 28 g/g to 60 g/g, or of from 30 g/g to 50 g/g, or of from 30 g/g to 40 g/g as measured according to the SAP K(t) test method disclosed herein. The SAP may have an effective permeability at 20 minutes (SAP K20) of at least $1 \times 10^{-8}$ cm$^2$, or at least $2 \times 10^{-8}$ cm$^2$, or at least $2.5 \cdot \times 10^{-8}$ cm$^2$, or of $3 \times 10^{-8}$ cm$^2$ to $1 \times 10^{-7}$ cm$^2$, or of $2 \times 10^{-8}$ cm$^2$ to $7 \times 10^{-8}$ cm$^2$, or of $2.5 \cdot \times 10^{-8}$ to $5 \times 10^{-8}$ cm$^2$ as measured according to the SAP K(t) test method.

The SAP may also have a ratio between the minimum effective permeability and the permeability at 20 minutes (SAP Kmin/SAP K20 ratio) of more than 0.75, or more than 0.8 or more than 0.85 as measured according to the SAP K(t) test method. In such embodiments the transient gel blocking is minimum and the liquid exudates are able to travel fast through the void spaces present between the particles throughout all the swelling process and especially in the initial part of the swelling phase which is the most critical for the first gush.

The superabsorbent polymer particles may further have a permeability at equilibrium expressed as UPM (Urine Permeability Measurement) value of more than 10, or preferably more than 15, or more than 20, or more than 30, or more than 45 or of 10 to 200, or of 15 to 100, or of 30 to 80 UPM units, where 1 UPM unit is $1 \times 10^{-7}$ (cm$^3$.s) /g. The UPM value is measured according to the UPM Test method described herein. This method is closely related to the SFC test method of the prior art. The UPM Test method typically measures the flow resistance of a preswollen layer of superabsorbent polymer particles, i.e. the flow resistance is measured at equilibrium. Therefore, such superabsorbent polymer particles having a high UPM value exhibit a high permeability when a significant volume of the absorbent article is already wetted by the liquid exudates. These embodiments exhibit good absorption properties not only at the first gush but also at the subsequent gushes.

The total amount of SAP present in the absorbent core may also vary according to expected user of the article. Diapers for newborns require less SAP than infant or adult incontinence diapers. The amount of SAP in the core may be for example comprised from about 2 to 50 g, in particular from 5 to 40 g for typical enfant diapers. The average SAP basis weight within the absorbent core may be for example of at least 50, 100, 200, 300, 400, 500 g/m$^2$ or more, or from 200 to 400 g/m$^2$.

### SAP vertical distribution

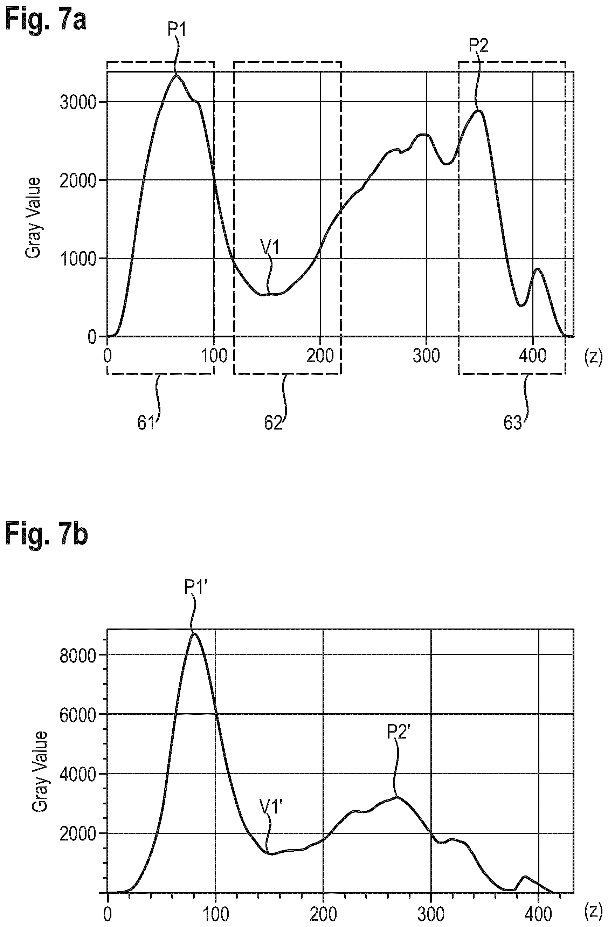

The SAP particles have preferably a heterogenous vertical distribution in the high loft central layer. By heterogenous it is meant that the planar SAP concentration in the z direction of the core is not constant through the thickness of the high loft nonwoven, but the SAP concentration varies in the z direction by more than plus or minus 10% relative to the average value, in particular by more than plus or minus 20%. The planar concentration as meant herein is the average planar concentration of a circular zone of the absorbent core of a diameter of 20 mm, in plane with the xy-direction of the absorbent core. The planar concentration of the SAP particles may in particular have a multi-modal distribution, in particular a bi-modal distribution, in the z-direction of the absorbent core. By bi-modal it is meant that the concentration of the SAP particles along the thickness of the core comprise at least two peaks, wherein the peaks are separated by a valley having a SAP concentration of less than 40%, in particular less than 30%, relative to the lowest concentration of the two neighboring peaks. Such a distribution is illustrated by the diagram in Fig. 7a and can be determined for a given sample by micro-CT analysis. By multi-modal, it is meant that the z-distribution of the concentration of the SAP particles comprises two or more of such peaks.

A bimodal distribution is illustrated in the Figs. 6-10, which were obtained by micro-CT analysis of an exemplary core according to the invention. CT is abbreviation of computer tomography, and "micro" means that a very low resolution can be reached, which is suitable for measuring the position of SAP particles. CT technology uses X-rays to see inside an object, with many X-ray projections taken around a specimen from different angles to produce a tomography image within the specimen. It is a conventional diagnose method in medical application. Today, the application areas of CT are diverse and extensive, as any material or component can be examined with CT. The major application areas of CT in science and industry are non-destructive testing.

Fig. 6a-c shows the results of a micro-CT scan of the absorbent core according to the invention detailed below in the example section. A circular sample having a diameter of 26 mm was cut from the middle of the absorbent core, with the field of view as seen in Fig. 6a being of 20 mm diameter in the center of the circular sample. The spatial resolution of the scan is 10 $\mu$m. The micro-CT scan allows the scanned data to be computer treated, and can be projected on the xz plane (Fig. 6b) and the yz plane (Fig. 6c). The bimodality of the distribution of the concentration of the SAP in the central layer is already visible in these Fig. 6a-c, with two distinct layers having a higher SAP concentration separated by a low SAP concentration zone. Using image analysis, a diagram showing the gray value which is proportional to the average planar concentration of SAP for the field of view of the sample in the z direction can be plotted - see Fig. 7a. The horizontal axis indicates the vertical distance z from the surface of the sample (100 = 1 mm), and the vertical axis shows the gray value which is proportional to the average planar concentration of the SAP for the reported distance z from the top surface. A relative difference in the gray value as depicted in Fig. 7a,b corresponds to the relative difference in the planar concentration of the SAP. For example, 20% higher gray value (as in Figs. 7a,b) corresponds to 20% higher planar concentration of SAP.

As can be seen from this diagram, the SAP distribution is bimodal with a first peak P1 present at approximately 0.6 mm from the top side of the core and having a gray value of 3338, and the second peak P2 at approximately 3.6 mm from the top side of the core at a value of 2897. Between the peaks P1 and P2 a valley V1 is present, having a value of 530. The gray values (as used in Fig. 7a) used for measuring the relative height of the peaks is an arbitrary unit which is not calibrated, but is directly correlated to the local density in the specimen and therefore directly correlated to the planar concentration of the SAP. Thus the diagram as in Fig. 7a can be used to compare the relative values of the peaks and valleys at different z values, i.e. at different location in the vertical axis of the core (thickness direction).

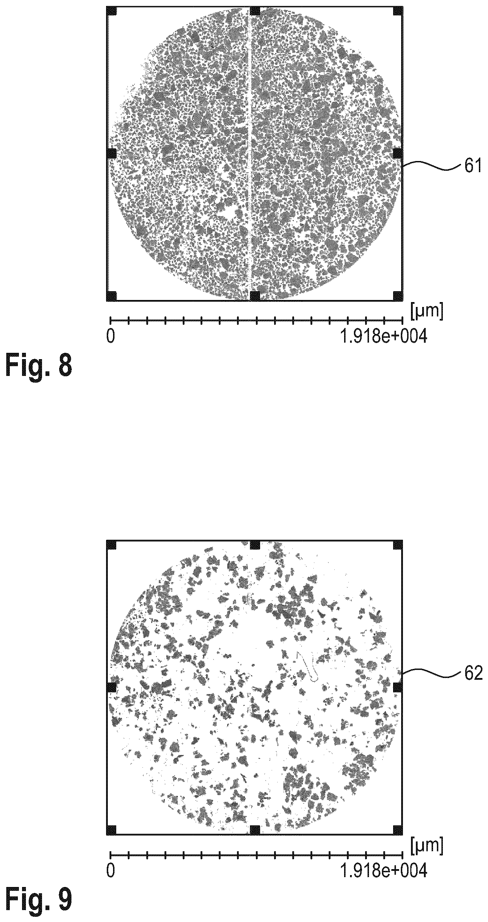

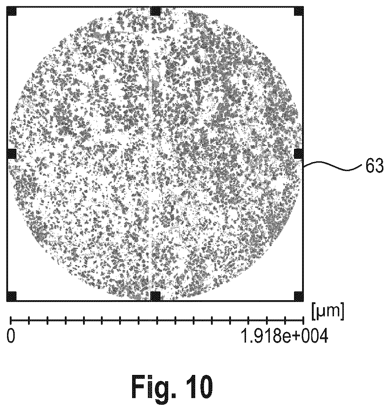

Fig. 8 is a view in the xy plane of the SAP present in a first section 61 encompassing the first 1 mm of the central layer, encompassing the first peak P1. Fig. 9 shows the SAP in a second 1 mm thick section 62 of the central layer in the region of the valley V1. Fig. 10 shows the SAP present in a third 1 mm thick section of the central layer, encompassing the second peak P2.

As indicated before, by bi-modal it is meant that the measure values for the valley (V1) is less than 40%, in particular less than 30%, than the lowest value of the two neighboring peaks (taking the lowest values for P1, P2). In the example shown, the valley V1 has a SAP concentration which is about 18.3% of the lowest of the neighboring peaks (530 / 2897 * 100%). By comparison, the distribution in the comparative example disclosed below had a first peak P1' at about 8400 arbitrary units, a second peak P2' at about 3000 arbitrary units, and a valley V1' in between at about 1500 arbitrary units, the valley being thus at about 50% of the lowest neighboring peak.

## Absorbent core properties

Instead or in addition to measuring the SAP T20 values of the SAP in the absorbent core, the K(t) test method may be adapted to measure the properties of the absorbent core directly. The properties of the core are measured on a circular sample taken in the center of the core, as discussed in detail in the Absorbent Core K(t) Test Method described further below.

In particular the T15 and permeability K20 of the absorbent core may thus be measured (Core T15, Core K20). Other parameters such as the Core T80%, which measures the uptake speed relative to the total capacity of the core, may also be measured.

The absorbent core of the invention may have a time to reach an uptake of 15 g/g (Core T15) of less than 200 s, in particular of less than 180 s or less than 150 s. The Core T15 may optionally be of at least 80 s, or 100 s, or 120 s, and any ranges constructed from any of these boundaries, for example from 100 to less than 180 s, or from 120 s to less than 150 s.

The core permeability (Core K20) may be more than $6.0 \times 10^{-8}$ cm$^2$, in particular more than $8.0 \times 10^{-8}$ cm$^2$, and for example from $6.0 \times 10^{-8}$ cm$^2$ up to $40 \times 10^{-8}$ cm$^2$, or from $8.0 \times 10^{-8}$ cm$^2$ up to $19 \times 10^{-8}$ cm$^2$, as measured according to Absorbent Core K(t) Test Method as described herein.

While not wishing to be bound by theory, the inventors believe that the low Core T15 value are characteristics of absorbent cores that can quickly absorb fluid not only into the void volume of the absorbent core (capillary storage) but also in the SAP particles that are comprised in the absorbent core (osmotic storage). The inventors believe that improving the balance of the capillary storage between the high loft fibers and the osmotic storage in the SAP provides absorbent cores that are fast in speed of acquisition of fluid insults, leading to superior leakage protection, and deliver comfort to the wearer by dryness. Fluid which is osmotically stored does not contribute to rewet, so leading to better dryness and better comfort for the wearer, and is also locally immobilized, so it leads to less fluid spreading as far in the longitudinal direction of the absorbent core as with other type of absorbent cores.

Furthermore, the osmotic capacity as expressed by CRC of the SAP particles comprised in the core, or more precisely the product of CRC of the SAP particles comprised in the core by the amount of the SAP particles in the absorbent core is not suitable to describe the ability of the core to efficiently balance the capillary versus the osmotic storage in the absorbent core of this invention.

The Absorbent Core K(t) Test Method as described herein measures the properties of the core under moderate pressure and dynamic behavior. The inventors have found that this balance can be reliable measured for high loft cores by the Absorbent Core K(t) Test Method. The inventors believe that the time to reach 15 g/g (Core T15) is characteristics for an absorbent core that has excellent balance between capillary storage and osmotic storage.

While not wishing to be bound by theory, the inventors believe that the high Core K20 values are characteristics of absorbent cores with high fluid permeability, especially in the swollen state. Such high core permeability in the swollen state ensures fluid intake into a loaded absorbent core, for example, subsequent liquid insults, even under moderate pressure. The inventors believe that the high Core K20 values characterize cores that have minimum liquid flow on top of the absorbent core. Avoiding this free liquid flow on top of the absorbent core limits the liquid spread in the top layers of an absorbent article in the longitudinal direction of the absorbent core as with other type of absorbent cores. This leads to better dryness and better comfort for the wearer.

Additional parameters may be measured using the Absorbent Core K(t) Test Method. The absorbent core of the invention may further have a time to reach 80% of the total uptake after 20 min (Core T80%) of less than 270 s, in particular of less than 260 s or less 255 s. The Core T80% may optionally be of at least 150 s, or 180 s, or 220 s, and any ranges constructed from any of these boundaries, for example from 150 to less than 270 s, or from 180 s to less than 260 s.

The absorbent core of the invention may have a time to reach an uptake of 20 g/g (Core T20) of less than 550 s, in particular of less than 500 s or less 450 s. The Core T20 may optionally be of at least 200 s, or 300 s, or 350 s, and any ranges constructed from any of these boundaries, for example from 300 to less than 550 s, or from 360 s to less than 500 s. The density of the absorbent core as a whole may also be measured when performing Absorbent Core K(t) Test Method. The absorbent core may have a density of from 0.2 $g/cm^3$ to less than 0.6 $g/cm^3$, in particular from 0.3 to less than 0.5 $g/cm^3$. The density is measured at 0.3 psi, as indicated in the Absorbent Core K(t) Test Method (see below).

## Process for making

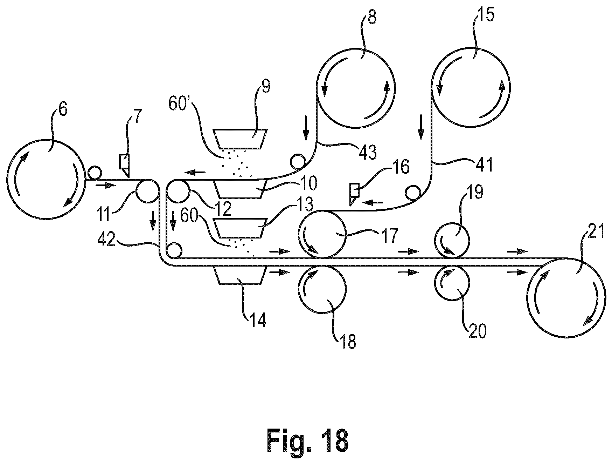

An exemplary continuous process for making the absorbent cores is illustrated in Fig. 18. The process and apparatus discussed above is generally similar to the one disclosed in CN101797201 or in Fig. 3 of WO2020/025401 (BASF, Ge et al.). The various arrows in this Figure represent the rotational directions of the various roll-releasing cylinders and roll-winding cylinders and the running directions of the manufacturing materials during the production flow process. Other processes and modifications are of course possible.

As illustrated in Fig. 18, the apparatus for making the absorbent cores may include a bottom layer web unwinder 6, a bottom layer glue spraying head 7, a high loft central layer web unwinder 8, a first SAP particles dispenser 9 and optional vacuum suction box 10, a first pair of rollers 11 and 12, a second SAP particles dispenser 13 and optional vacuum suction box 14, a top layer web unwinder 15, a top layer spraying head 16, second pair of rollers 17 and 18, trimming off knives 19, 20, and a product roll winding roller 21.

The first and second SAP particles dispenser 9, 13 may be both provided with a frequency changing and speed adjusting device (not drawn in Figure 18), adjusted to maintain a vibration frequency that matches the product roll winding roller 21 linear velocity and to ascertain that the deposited SAP is mostly uniformly distributed on the high loft web 43.

During production, a roll of bottom layer material 42, for example a paper or nonwoven roll, is installed on the bottom layer web unwinder 6. A high loft nonwoven fabric roll 43 is installed on the central layer web unwinder 8. The initial density and thickness of the high loft central layer may be conveniently measured on the raw material Thickness and Density Measurement Method further described below.

The SAP particles are charged in the first and second SAP particles sieve plates 9 and 13. A roll of top layer material 41, which can be a paper or nonwoven roll, is installed on the top layer web unwinder 15. During the continuous process of making the absorbent cores, the bottom layer 42 passes through the spraying head 7 and is applied on one side with a glue 72, before being attached to the central layer 43 between the first press rollers 11 and 12. The high loft nonwoven central layer 43 passes through the first SAP dispenser 9 and vacuum suction box 10, wherein the SAP particles 60' are deposited into the central layer and at least partially distributed into the fibers the central layer from a first side. It is also possible that the bottom layer material 6 is first attached to on a first side of the central layer 43 before the SAP particles 60 are deposited onto and blended between the fibers of the central layer.

After the bottom layer 42 and the central layer 43 have been pressed together between the rollers 11 and 12, these combined layers may optionally pass between a second SAP particles sieve plate 13 and vacuum suction box 14 that cooperate to deposit SAP particles onto the second surface of the central layer and blend the SAP particles in the fibers of the central layer from this second surface. The top layer 41 which has been applied with an adhesive 72 by a glue spraying head 16 is then joined to the central layer to cover the second surface of the central layer between two press rollers 17 and 18. Of course in the preceding the top layer and bottom layer may be used interchangeably.

The press rollers 17 and 18 may have a substantially flat surface, or they may have elevated areas where extra pressure and heat should be applied onto the core. These elevated areas may coincide with the channel areas, and thus provide a mechanical bonding, ultrasonic bonding and/or heat bonding within the channel zones 26. The press rollers 11-12, 17-18 may be heated. It is also possible that the rollers have elevated areas along the longitudinal side edges and/or the back and front edges (360° perimeter) the core. A better bonding can be achieved in these zones when they are free of SAP as in the channel zones 26. Trimming knives 19 and 20 can be provided to trim the longitudinal side edges of the continuous band of absorbent core before the stream of the absorbent core material is finally rolled into a roll of absorbent core material by the product roll winding roller 21.

The roll of absorbent core material thus formed may be stored or transported to an article production site where it is further converted into an absorbent product. It is also possible that instead of forming a roll, the stream of absorbent core material may be directly fed into a converting line, in which case the absorbent cores will be individualized by cutting along their front and back edges.

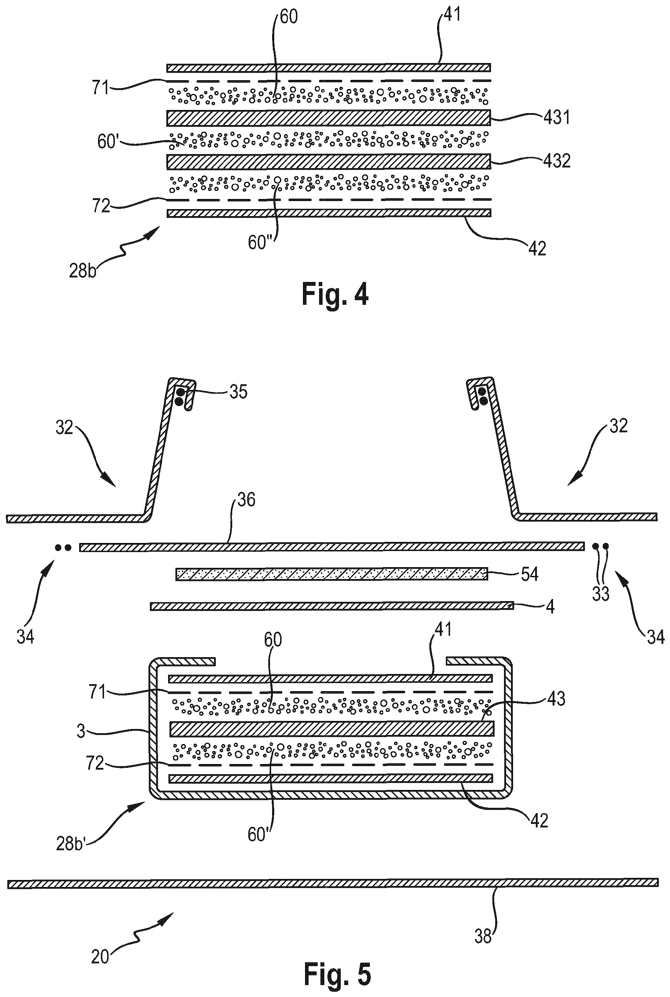

A wrapping layer 3 (not represented in Fig. 18) may also be fed before the core material is rolled to wrap the top, central and bottom layer as shown discussed in relation to Fig. 5 to prevent losses of SAP though the side edges of the absorbent core. Alternative such wrapping layer may also be attached to the core when further converting the core material web.

## Cores 28b with dual high loft nonwoven layers 431, 432

The absorbent cores 28 discussed previously comprise a single high loft nonwoven layer, however it is also possible that the absorbent cores comprise two (or more) high loft nonwoven layers between the top and bottom layers. This is illustrated for example in Fig. 4, wherein an absorbent core 28b comprising a first central layer 431 and the second central layer 432 are shown sandwiched between the top layer 41 and the bottom layer 42.

The absorbent core 28b thus may comprise a first central layer 431 and a second central layer 432, each of which being a high loft fibrous nonwoven layer comprising for example three or more superabsorbent polymer particle layers 60, 60', 60" at least partially distributed within the pores of the high loft central layers. The two (or more) high loft central layers may be comprised of the same material or different high loft nonwovens. For example, the permeability in the upper central layer may be enhanced by using a low basis weight high loft and softness may be enhanced in the bottom layer with a denser high loft material. Of course, other configurations are also possible. The two or more two central layers can be of equal dimensions in the x,y plane of the core, but they may also have different length and/or width. Two central layers of unequal length could be beneficial to provide different amount of SAP along the absorbent core, and made for example by adding a cut and slit unit on the second layer patch, and before combining it with the first layer.

It is also considered that the two high loft central layers may comprise different sort of SAP, for example faster absorbing SAP (lower SAP T20, higher UPM value) deposited in a first SAP layer 60 on the top side of the first central layer 431 closer to the top layer 41 and/or a more absorbing SAP (higher CRC) in a second SAP layer 60' or a third SAP layer 60" which is at least partially distributed within the second central layer 432, closer to the bottom layer 42. The SAPs in the two high loft layers can also vary in absorption speed (SAP T20), in capacity (CRC, AAP) and permeability (UPM), or alternatively be the same.

Absorbent cores comprising a dual high loft nonwoven layers may be made by a method adapted from one of the methods disclosed above, see for example WO2016/106021A1 where two separate high loft layer releasing cylinders are described to provide for the first and second high loft central layers 431, 432. As an alternative, a high loft web having a double width may be used: such a large width roll can be cut after release in machine direction in two halves providing for two streams of high loft nonwoven material that are then separately deposited with SAP particles. The two streams of high loft material 431, 432 may be then combined separately with the top layer and bottom layer respectively, each having then SAP particles 60 deposited onto them through a suitable SAP deposition device.

## Absorbent article 20

The absorbent cores may be incorporated into any kind of personal hygiene articles, in particular pant diapers and taped diapers, as well as inserts in hybrid systems comprising a washable outer cover and a disposable insert. A schematic cross-sectional view showing some of the main components of a diaper absorbent article 20 is illustrated in Fig. 5. In this Figure, the absorbent core of Fig. 3 (with a wrapping layer 3) is shown, but this is of course not limiting and for illustration only. Absorbent articles typically comprise a wearer-facing fluid permeable topsheet 36 and a garment-facing liquid impermeable backsheet 38 attached to each other along their perimeter. The absorbent core is placed between these layers and may be attached directly and indirectly to these layers, typically by gluing or heat/pressure bonding.

The topsheet 36 is preferably compliant, soft-feeling, and non-irritating to the wearer's skin. Further, at least a portion of the topsheet is liquid permeable, permitting liquids to readily penetrate through its thickness. A suitable topsheet may be manufactured from a wide range of materials, such as porous foams, reticulated foams, apertured plastic films, or woven or nonwoven materials of natural fibers (e.g., wood or cotton fibers or viscose), synthetic fibers or filaments (e.g., polyester or polypropylene or bicomponent PE/PP fibers or mixtures thereof), or a combination of natural and synthetic fibers. If the topsheet includes fibers, the fibers may be spunbond, carded, wet-laid, meltblown, hydroentangled, or otherwise processed as is known in the art, in particular spunbond PP nonwoven. Typical diaper topsheets have a basis weight of from about 10 gsm to about 28 gsm, in particular between from about 12 gsm to about 18 gsm but other basis weights are possible.

The backsheet 38 is typically impermeable to liquids (e.g. urine). The backsheet may for example be or comprise a thin plastic film such as a thermoplastic film having a thickness of less than about 0.10 mm. Exemplary backsheet films include those manufactured by Tredegar Corporation, based in Richmond, VA, and sold under the trade name CPC2 film. Other suitable backsheet materials may include breathable materials which permit vapors to escape from the article while still preventing exudates from passing through the backsheet. A covering low basis weight nonwoven may be attached to the external surface of the film to provide for a softer touch.

The absorbent articles may also comprise a liquid management layer 54 (also called fluid acquisition or fluid distribution layer) directly under the topsheet 36. The function of such a layer is to rapidly acquire the fluid from the topsheet away from the wearer-facing side and/or to distribute over a larger area so it is more efficiently absorbed by the absorbent core. It is also possible that such a liquid management layer may be placed between the backsheet and the absorbent core. A further layer 4 may be present between the liquid management 54 and the absorbent core 28. The further layer 4 may be another such acquisition or distribution layer, or may be a tissue paper or low basis weight NW layer that provides an additional wrapping of the absorbent core 28 to avoid SAP particles from escaping outside the core.

Absorbent articles such as diapers or training pants may typically further comprise components that improve the fit of the article around the legs of the wearer, in particular barrier leg cuffs 32 and gasketing cuffs 34. The barrier leg cuffs may be formed by a piece of material, typically a nonwoven, which is partially bonded to the rest of the article and can be partially raised away and thus stand up from the plane defined by the topsheet. The barrier leg cuffs are typically delimited by a proximal edge joined to the rest of the article, typically the topsheet and/or the backsheet, and a free terminal edge intended to contact and form a seal with the wearer's skin. The standing up portion of the cuffs typically comprise an elastic element, for example one or a plurality of elastic strands 35. The barrier leg cuffs provide improved containment of liquids and other body exudates approximately at the junction of the torso and legs of the wearer.

In addition to the barrier leg cuffs, the article may comprise gasketing cuffs 34, which are formed in the same plane as the chassis of the absorbent article, in particular which may be at least partially enclosed between the topsheet or the barrier leg cuffs and the backsheet, and may be placed laterally outwardly relative to the upstanding barrier leg cuffs. The gasketing cuffs can provide a better seal around the thighs of the wearer. Usually each gasketing leg cuff will comprise one or more elastic string or elastic element 33 comprised in the chassis of the diaper for example between the topsheet and backsheet in the area of the leg openings.

The absorbent articles may also include other typical components found in diapers, training pants, replaceable inserts or adult incontinence products (and not further represented). A releasable fastening system for taped diapers may be provided to provide lateral tensions about the circumference of the absorbent article to hold the absorbent article on the wearer. This fastening system is not necessary for training pants since the waist region of these articles is already bonded. The fastening system usually comprises a fastener such as tape tabs, hook and loop fastening components, interlocking fasteners such as tabs & slots, buckles, buttons, snaps, and/or hermaphroditic fastening components, although any other known fastening means are generally acceptable. A landing zone is normally provided on the front waist region of the article for the fastener to be releasably attached.

The absorbent article may comprise front ears and back ears as is known in the art. The ears can be integral part of the chassis, for example formed from the topsheet and/or backsheet as side panel. Alternatively, they may be separate elements attached by gluing and / or heat embossing. The back ears are advantageously stretchable to facilitate the attachment of the tabs on the landing zone and maintain the taped diapers in place around the wearer's waist. The front ears may also be elastic or extensible to provide a more comfortable and contouring fit by initially conformably fitting the absorbent article to the wearer and sustaining this fit throughout the time of wear well past when absorbent article has been loaded with exudates since the elasticized ears allow the sides of the absorbent article to expand and contract.

Typically, adjacent layers will be joined together using conventional bonding method such as adhesive coating via slot coating or spraying on the whole or part of the surface of the layer, or thermo-bonding, or pressure bonding or combinations thereof. The bonding between components is for clarity and readability not represented in the majority of Figures, in particular Fig. 5, except for adhesive layers 71, 72. Adjacent layers of the article should be considered to be attached to another unless specifically mentioned otherwise. For example the backsheet and the bottom layer of the absorbent core may be typically glued together. The adhesives used may be any standard hotmelt glue as known in the art.

## Packaging

The absorbent articles may be packaged in any type of conventional packaging. The absorbent articles may be in particular compressed when packaged to save space. In particular the package may comprise a plurality of the absorbent articles, wherein the package has an in-bag stack height of less than about 80 mm, according to the In-Bag Stack Height Test as described in US 8,585,666 B2 (Weisman). Alternatively, packages of the absorbent articles of the present disclosure may have an in-bag stack height of from about 72 mm to about 80 mm or from about 74 mm to about 78 mm, specifically reciting all 0.5 mm increments within the specified ranges and all ranges formed therein or thereby, according to the In-Back Stack Height Test as described in US 8,585,666 B2 (Weisman).

## Examples and experimental results

a) Core construction

Exemplary absorbent cores according to the invention were hand-made. The central high loft layer was a high loft layer (TL6 from TWE-group, Emsdetten, Germany). The measured caliper was 0.613 mm and the basis weight 85.4 $g/m^2$ (average of 10 measurements), giving a density of about 0.140 $g/cm^3$, measured at a pressure of 4.14 kPa (0.6 psi). The data at different pressures are listed in the table below.

Table 1

|  | 4.14 kPa (0.6 psi) | 2.07 kPa (0.3 psi) | 0.83 kPa (0.12 psi) |
|---|---|---|---|
| Caliper (mm) | 0.613 | 0.681 | 0.900 |
| Density ($g/cm^3$) | 0.140 | 0.128 | 0.099 |
| Basis weight ($g/m^2$) | 85.4 | 86.8 | 87.5 |

The two cover layers were hydrophilic 10 gsm SMS nonwoven from Fibertex (for example HY02XXX10). The width of the core layers was 165 mm and its length 360 mm. The cover layers were each applied first with a 5 gsm spiral glue, and then a 10 gsm net of microfiber glue (NW1151ZP ex. FULLER ADHESIVES) uniformly over their whole length.
The high loft central layer was placed on the bottom cover layer with the rough side of the high loft nonwoven facing up. The width of the high loft central layer was 110 mm and its length 360 mm. 15 g of the SAP was homogenously hand sprinkling over the surface of the high loft of the nonwoven. Because the making table did not have a vacuum, the SAP were distributed in the high loft nonwoven by hand-brushing of the SAP particles. The top cover layer was then placed over the laminate with the glue layer facing the high loft layer. Gentle pressure was applied with a rubber roller to attach the central layer with the two cover layers.
The resulting basis weight of SAP in the absorbent core was 380 gsm. The average concentration of SAP in the absorbent core was 61% by weight of the core, the average concentration of SAP relative to the high loft central layer alone was 81% by weight (not taking into account the top and bottom layers and glue).
Two commercially available SAPs (SAP E1, SAP C1) were used to make an inventive core E1 and a comparative core C1, respectively. The SAPs had the properties as listed in Table 2 below.

Table 2: Properties of SAPs.

|  | SAP E1 | Comparative SAP C1 |
|---|---|---|
| SAP name | HVDE 235 "Der Alleskoenner" ex. Schauch | Sodium Polyacrylate ex. Alquera (alquera.com) |
| SAP T20 (s) | 165 | 312 |
| AAP@0.7 psi (g/g) | 23.5 | 21.7 |
| AAP@0.3 psi (g/g) | 28.9 | 29.8 |
| CRC (g/g) | 27.0 | 33.3 |
| UPM ($10^{-7}$ $cm^3$ s/g) | 46 | 3 |
| SAP U20 [g/g] | 31.1 | 31.4 |
| SAP K20 [$10^{-8}$ $cm^2$] | 2.5 | 0.8 |
| SAP Kmin/SAP Kmax | 0.88 | 0.85 |
| SAP T10 (s) | 63 | 109 |

The absorbent cores had the following properties:

Table 3: Properties of absorbent cores E1 and C1.

|  | Inv. Core E1 | Comparative Core C1 |
|---|---|---|
| SAP used | 15 g of SAP E1 | 15 g of SAP C1 |
| Specific Average Density $\rho_s$ (g/cm$^3$) | 1.52 | 1.50 |
| Core Density (at 0.3 psi) $\rho_{core}$ (g/cm$^3$) | 0.40 | 0.35 |
| Core T20 (s) | 356 | 585 |
| Core T15 (s) | 143 | 265 |
| Core T80% (s) | 253 | 464 |
| Core U20 (g/g) | 23.1 | 23.4 |
| Core K20 ($10^{-8}$ cm$^2$) | 20.3 | 6.0 |

b) Comparative Commercial cores:

As further comparison, the properties of two commercial products in China: Teddy Bear, More Than Thinner, size S and Huggies, breathable diaper, size L were tested according to the Absorbent Core K(t) Test Method. Teddy Bear had an absorbent core with a high loft central layer in which SAP was distributed. Huggies had a dual layer core comprising an airfelt/SAP mix layer above a high loft layer with SAP, with both layers between a bottom layer and top layer. The Specific Average Density $\rho_s$ of the core samples was not measured but assumed to be 1.50 g/cm$^3$.
The properties of the comparative cores C2 and C3 are listed in table 4.

Table 4: Properties of comparative absorbent cores C2 and C3.

|  | Teddy Bear, size S | Huggies, breathable diapers, size L |
|---|---|---|
| Comparative Core | C2 | C3 |
| Assumed Specific Average Density $\rho_s$ (g/cm$^3$) | 1.50 | 1.50 |
| Number of replicates measured | n = 4 | n = 3 |
| Core Density at 0.3 psi $\rho_{core}$ (g/cm$^3$) | 0.25 | 0.22 |
| Core T20 (s) | 799 | 956 |
| Core T15 (s) | 223 | 202 |
| Core T80% (s) | 318 | 277 |
| Core U20 (g/g) | 20.9 | 20.4 |
| Core K20 ($10^{-8}$ cm$^2$) | 3.9 | 5.7 |

The SAP used in these products were collected and also measured using the SAP K(t) Test Method, this time assuming an Assumed Specific Average Density $\rho_s$ for the SAP of 1.60 g/cm$^3$.
The properties of the SAPs of the comparative cores, SAP C2 and SAP C3 are listed in table 4.

Table 5: Properties of the SAPs of the comparative cores, SAP C2 and SAP C3.

|  | Teddy Bear, size S | Huggies, breathable diapers, size L |
|---|---|---|
| AGM | SAP C2 | SAP C3 |
| Assumed Specific Average Density $\rho_s$ | 1.60 g/cm$^3$ | 1.60 g/cm$^3$ |
| Number of replicates measured | n = 1 | n = 3 |
| SAP T20 (s) | > 1000 | 307 |
| SAP K20 ($10^{-8}$ cm$^2$) | 0.0 | 0.2 |
| CRC (g/g) | - | 30.0 |
| AAP@0.7 psi (g/g) | - | 20.2 |

(continued)

| | Teddy Bear, size S | Huggies, breathable diapers, size L |
|---|---|---|
| UPM ($10^{-7}$ cm$^3$ s/g) | - | 2 |

c) Product data: Speed of Acquisition

The exemplary core E1 and the comparative core C1 were placed in an absorbent product chassis comprising a commercial topsheet, backsheet and acquisition layer, as used in the Pampers Premium product in China (Size 4). The absorbent cores were placed in the product with different orientation (up or down - see explanation below). These products were tested using the C-SABAP tests (Curved-Speed of Acquisition with Balloon Applied Pressure). The C-SABAP determines saline acquisition times of absorbent hygiene products, while the diaper is held in a slightly curved position and placed on a latex film which is inflated by pressurized air (2.07 kPa (0.30 psi)) and monitored by a digital manometer.

In a first set of experiment, the speed of acquisition was measured on 4 replicates for each type of core and for each orientation of the core in the diaper. Four gushes of each 75 ml of physiological colored saline (0.9 w.%) were applied sequentially at a rate 15 ml/s, with 5 minutes between each gush. The speed of acquisition for each gush is recorded. The liquid is delivered in the center of the diaper at a distance of 170 mm from the front of the absorbent core. The table below gives the average of 3 measurements out of 4 for each core/orientation combinations, the 4th measurement with the highest sum of gush time 1 & 2 was disregarded to eliminate outliers.

Table 6

| Core Orientation | Core E1 up | Core E1 down | Comp. Core C1 up | Comp. Core C1 down |
|---|---|---|---|---|
| | AVG 3 of 4 | AVG 3 of 4 | AVG 3 of 4 | AVG 3 of 4 |
| 1st gush [s] | 41 | 37 | 63 | 53 |
| 2nd gush [s] | 126 | 94 | 190 | 116 |
| 3rd gush [s] | 198 | 172 | 269 | 170 |
| 4th gush [s] | 326 | 222 | 366 | 226 |
| Note: "up" means that the top side of the central layer on which the AGM was added is facing towards the topsheet, and "down" means the opposite orientation. | | | | |

The speed of acquisition of the inventive example E1 was faster for the 1st gush, independent from orientation (up or down) of the core in the product compared with the comparative example C1.

At same orientation (either all up or all down), the inventive cores E1 performed better in speed of acquisition (i.e. faster) across all 4 gushes vs. the products with the comparative core C1.

d) Product data: Liquid distribution and Rewet

The articles which were used for C-SABAP test were removed five minutes after complete absorption of the last fourth gush from the C-SABAP apparatus. The Liquid distribution and Rewet was measured on these articles comprising the cores of interest. The Rewet was measured at the point of insult, i.e. at a distance of 170 mm from the front of the absorbent core. The Liquid distribution was measured in longitudinal direction (y) starting from the center point of the insult (i.e. at a distance of 170 mm from the front of the absorbent core) towards each transversal side of the article on a planar surface, resulting in Liquid Distribution Front, i.e. from insult to the front end of the absorbent core and in Liquid Distribution Back, i.e. from insult to the back end of the absorbent core.

The liquid distribution measures the length of the stain left by the colored saline on the absorbent core at the front and back of the diaper (the edge of the colored stain is not a straight line so that the maximum and minimum distance between stain edge and loading point is indicated).

A rewet test was then conducted on the topsheet side of the diaper. The Overall Liquid Distribution Length is the sum of the averages of the average values of the Min and Max values of the both sides, i.e. Average(Front Min, Front Max) plus Average (Back Min, Back, Max).

The rewet test uses a skin like material (stack of 5 layers of collagen) to measure the amount of liquid which is released by the diaper. Collagen absorbs liquid by the same mechanism as babies skin does. The rewet test is performed 10 minutes after the last gush, with four 70 mm diameter collagen sheets stacked centered at the gush point and weighted with a 9.1 kg weight for 30 s. The amount of fluid absorbed on the stack of collagen sheets is measured and reported as indicated below.

As outline above, the data of the product that was excluded in Speed of Acquisition, is also excluded in Rewet and Liquid Distribution, hence table 5 give average of 3 data points out of 4.

Table 7

| | Inv. Ex. E1 up | Inv. Ex. E1 down | Comp. Ex. C1 up | Comp. Ex. C1 down |
|---|---|---|---|---|
| Front Min [mm] | 153 | 138 | 170[1] | 148 |
| Front Max [mm] | 153 | 138 | 170[1] | 162 |
| Back Min [mm] | 142 | 143 | 152 | 157 |
| Back Max [mm] | 142 | 143 | 165 | 170 |
| Overall liquid distribution length [mm] | 295 | 282 | 328 | 318 |
| Rewet [mg] | 123 | 132 | 115 | 133 |

[1] 170 mm is the maximum value for Liquid Distribution length Front, meaning the liquid reached the front side of the absorbent core.

The stains on the inventive diaper with the inventive core E1 were significantly shorter than for the comparative diaper with the comparative core C1. For both diapers the Rewet was lower with the absorbent core placed with the top layer placed towards the topsheet ("up") than the opposite ("down").

e) microCT-scan

Exemplary core E1 was submitted to a micro-CT secan according the micro-CT Scan Method described below. The results of the microCT scan for the exemplary core E1 are as shown on Figs. 6-10. As explained before, such a CT scan can be used to determine the distribution of the concentration of SAP within the absorbent core, in particular determine if the vertical distribution of the SAP particles is homogeneous or bi-modal or multimodal. The inventive core E1 had a bi-modal distribution. The comparative core C1 was also measured and also comprised two peaks but is not considered bi-modal according to the definition herein (valley having a measured gray value of less than 40% than the lowest of the flanking peaks, in relative units). Although the SAP was not applied on both side of the high loft layer, it was found that the combination of material in the example used still resulted in a bi-modal SAP distribution, which would also be found when the SAP is applied in two steps sequentially for each side of the central layer.

Test procedures

**Centrifuge Retention Capacity (CRC)**

The CRC measures the capacity of the superabsorbent polymer particles to absorb for free swelling in excess liquid. The CRC is measured according to EDANA method NWSP 241.0.R2 (19).

**Absorption Against Pressure**

The AAP is measured according to EDANA standard test NWSP 242.0 R2 (19), with the pressure used being 0.7 psi and 0.3 psi, as indicated as AAP@0.7psi and AAP@0.3psi, respectively.

**Thickness and Density Measurement Method**

This method is used to measure the thickness (caliper) of the high loft central layer in a standardized manner. The density can then be calculated from the thickness and the basis weight of the layer. Unless otherwise mentioned, the thickness and density are indicated for the high loft material in the absence of SAP particles. The measurement should preferably be made on the high loft material before it was converted into an absorbent core and thus free of SAP. If the starting material is not available, the high loft central layer can be obtained by carefully extracting it from an absorbent core, and removing the majority of SAP particles for example by careful shaking or suction. A freeze spray may be used to separate the central layer from the other layers. The samples should be kept at least 24 hours at 21°C $\pm$ 2°C and 50% $\pm$

10% RH to equilibrate, in particular if they have been previously compressed.

Equipment: Mitutoyo manual caliper gauge with a resolution of 0.01 mm, or equivalent instrument.

Contact Foot: Flat circular foot with a diameter of 16.0 mm (± 0.2 mm). A circular weight may be applied to the foot (e.g., a weight with a slot to facilitate application around the instrument shaft) to achieve the target weight. The total weight of foot and added weight (including shaft) is selected to provide 4.14kPa of pressure (0.6 psi) to the sample.

In addition, the thickness can be determined at different pressures, using accordingly different weights applied to the foot. The applied pressure is indicated at the thickness and density, for example measured at 4.14 kPa (0.6 psi).

The caliper gauge is mounted with the lower surface of the contact foot in a horizontal plane so that the lower surface of the contact foot contacts the center of the flat horizontal upper surface of a base plate approximately 20 x 25 cm. The gauge is set to read zero with the contact foot resting on the base plate.

Ruler: Calibrated metal ruler graduated in mm.

Stopwatch: Accuracy 1 second.

Sample preparation: The central layer is conditioned at least 24 hours as indicated above.

Measurement procedure: The layer is laid flat with the bottom side, i.e. the side intended to be placed towards the backsheet in the finished article facing down. The point of measurement, i.e. the middle of the sample, is carefully drawn on the top side of the layer, taking care not to compress or deform the layer. In the unlikely case that the high loft nonwoven layer is not homogeneous in the transversal direction or longitudinal direction, the values are measured in the center of a sample corresponding to the center of an absorbent core that would be made from the sample.

The contact foot of the caliper gauge is raised and the central layer is placed flat on the base plate of the caliper gauge with the top side of the core up so that when lowered, the center of the foot is on the marked measuring point.

The foot is gently lowered onto the sample and released (ensure calibration to "0" prior to the start of the measurement). The caliper value is read to the nearest 0.01 mm, 10 seconds after the foot is released.

The procedure is repeated for each measuring point. Ten samples are measured in this manner for a given material and the average thickness is calculated and reported with an accuracy of one tenth mm. The basis weight of each sample is calculated by dividing the weight of each sample by their area.

The density, in $g/cm^3$, is calculated by dividing the basis weight (in $g/cm^2$) of the material by the thickness (in cm).

## Micro-CT Scan Method

A 44 mm diameter circular sample is carefully cut from the center of an absorbent core and placed in an adapted micro-CT apparatus sample holder. The field of view is reduced to the innermost circle having a diameter of 20 mm to avoid any influences of the cutting on the structure of the core. The CT scan machine used is for example a DynaTOM, manufactured by XRE nv (merged by Tescan), Serie number M2090. Settings were as follows: Tube voltage : 80 KV, Tube power : 10.0 Watt, Exposure time (ms) : 380, Number of averages : 1.000000, Binning value : 1, Voxel size : 10 $\mu$m, scan rate 2.36 frame per second, 2900 projection images totally for one 3D.

Further software that can be used to exploit the data collected are for example:

Image acquisition: Acquila developed by XRE nv, version 11.06.2019.

Image reconstruction: XRE Reco Version 1.0.0.117 developed by XRE nv.

Image post processing: Avizo 2019.1 by Thermo Fischer/ ImageJ 1.52p open source/ MS Excel 2016.

The spatial resolution of the scan is 10 $\mu$m. The micro-CT scan allows the scanned data to be computer treated and can be projected on the xz plane (Fig. 6b) and the yz plane (Fig. 6c). Using image analysis, a diagram showing the gray value which is proportional to the average planar concentration of SAP for the field of view of the sample in the z direction can be plotted - see e.g. Fig. 7a/7b. The horizontal axis indicates the vertical distance z from the surface of the sample (100 = 1 mm), and the vertical axis shows the gray value which is proportional to the average planar concentration of SAP for the field of view (circular with 20 mm diameter) for the reported distance z from the top surface.

## Urine Permeability Measurement (UPM) Test Method

### Lab Conditions:

This test has to be performed in a climate conditioned room at standard conditions of 23°C ± 2°C temperature and 45% ± 10% relative humidity.

### Urine Permeability Measurement System

This method determined the permeability of a swollen hydrogel layer 1318. The equipment used for this method is described below.

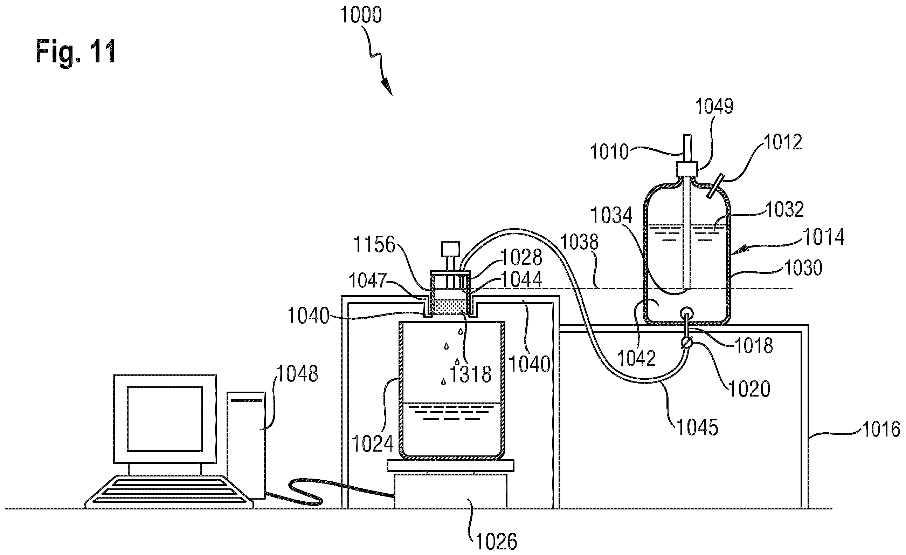

Fig. 11 shows permeability measurement system 1000 set-up with the constant hydrostatic head reservoir 1014, open-ended tube for air admittance 1010, stoppered vent for refilling 1012, laboratory rack 1016, delivery tube 1018 with flexible tube 1045 with Tygon tube nozzle 1044, stopcock 1020, cover plate 1047 and supporting ring 1040, receiving vessel 1024, balance 1026 and piston/cylinder assembly 1028.

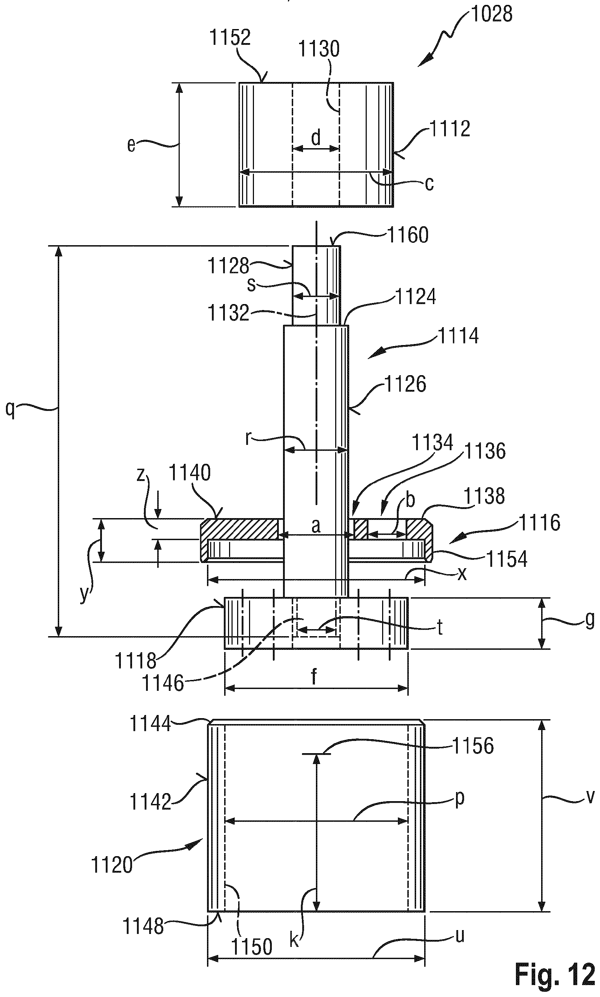

Fig. 12 shows the piston/cylinder assembly 1028 comprising a metal weight 1112, piston shaft 1114, piston head 1118, lid 1116, and cylinder 1120. The cylinder 1120 is made of transparent polycarbonate (e.g., Lexan®) and has an inner diameter p of 6.00 cm (area = 28.27 cm$^2$) with inner cylinder walls 1150 which are smooth. The bottom 1148 of the cylinder 1120 is faced with a stainless-steel screen cloth (ISO 9044 Material 1.4401, mesh size 0.038 mm, wire diameter 0.025 mm) (not shown) that is bi-axially stretched to tautness prior to attachment to the bottom 1148 of the cylinder 1120. The piston shaft 1114 is made of transparent polycarbonate (e.g., Lexan®) and has an overall length q of approximately 127 mm. A middle portion 1126 of the piston shaft 1114 has a diameter r of 22.15 ($\pm$ 0.02) mm. An upper portion 1128 of the piston shaft 1114 has a diameter s of 15.8 mm, forming a shoulder 1124. A lower portion 1146 of the piston shaft 1114 has a diameter t of approximately 5/8 inch (15.9 mm) and is threaded to screw firmly into the center hole 1218 (see Fig. 8) of the piston head 1118. The piston head 1118 is perforated, made of transparent polycarbonate (e.g., Lexan®), and is also screened with a stretched stainless-steel screen cloth (ISO 9044 Material 1.4401, mesh size 0.038 mm, wire diameter 0.025 mm) (not shown). The weight 1112 is stainless steel, has a center bore 1130, slides onto the upper portion 1128 of piston shaft 1114 and rests on the shoulder 1124. The combined weight of the piston head 1118, piston shaft 1114 and weight 1112 is 596 g ($\pm$ 6 g), which corresponds to 0.30 psi over the inner area of the cylinder 1120. The combined weight may be adjusted by drilling a blind hole down a central axis 1132 of the piston shaft 1114 to remove material and/or provide a cavity to add weight. The cylinder lid 1116 has a first lid opening 1134 in its center for vertically aligning the piston shaft 1114 and a second lid opening 1136 near the edge 1138 for introducing fluid from the constant hydrostatic head reservoir 1014 into the cylinder 1120.

A first linear index mark (not shown) is scribed radially along the upper surface 1152 of the weight 1112, the first linear index mark being transverse to the central axis 1132 of the piston shaft 1114. A corresponding second linear index mark (not shown) is scribed radially along the top surface 1160 of the piston shaft 1114, the second linear index mark being transverse to the central axis 1132 of the piston shaft 1114. A corresponding third linear index mark (not shown) is scribed along the middle portion 1126 of the piston shaft 1114, the third linear index mark being parallel with the central axis 1132 of the piston shaft 1114. A corresponding fourth linear index mark (not shown) is scribed radially along the upper surface 1140 of the cylinder lid 1116, the fourth linear index mark being transverse to the central axis 1132 of the piston shaft 1114. Further, a corresponding fifth linear index mark (not shown) is scribed along a lip 1154 of the cylinder lid 1116, the fifth linear index mark being parallel with the central axis 1132 of the piston shaft 1114. A corresponding sixth linear index mark (not shown) is scribed along the outer cylinder wall 1142, the sixth linear index mark being parallel with the central axis 1132 of the piston shaft 1114. Alignment of the first, second, third, fourth, fifth, and sixth linear index marks allows for the weight 1112, piston shaft 1114, cylinder lid 1116, and cylinder 1120 to be repositioned with the same orientation relative to one another for each measurement.

The cylinder 1120 specification details are:

Outer diameter u of the Cylinder 1120: 70.35 mm ($\pm$0.05 mm)
Inner diameter p of the Cylinder 1120: 60.0 mm ($\pm$0.05 mm)
Height v of the Cylinder 1120: 60.5 mm. Cylinder height must not be lower than 55.0 mm!

The cylinder lid 1116 specification details are:

Outer diameter w of cylinder lid 1116: 76.05 mm ($\pm$0.05 mm)
Inner diameter x of cylinder lid 1116: 70.5 mm ($\pm$0.05 mm)
Thickness y of cylinder lid 1116 including lip 1154: 12.7 mm
Thickness z of cylinder lid 1116 without lip 1154: 6.35 mm
Diameter a of first lid opening 1134: 22.25 mm ($\pm$0.02 mm)
Diameter b of second lid opening 1136: 12.7 mm ($\pm$0.1 mm)
Distance between centers of first and second lid openings 1134 and 1136: 23.5 mm

The weight 1112 specification details are:

Outer diameter c: 50.0 mm
Diameter d of center bore 1130: 16.0 mm
Height e: 39.0 mm

The piston head 1118 specification details are:

Diameter f: 59.7 mm ($\pm$0.05 mm)

Height g: 16.5 mm. Piston head height must not be less than 15.0 mm.

Outer holes 1214 (14 total) with a 9.30 ($\pm$0.25) mm diameter h, outer holes 1214 equally spaced with centers being 23.9 mm from the center of center hole 1218.

Inner holes 1216 (7 total) with a 9.30 ($\pm$0.25) mm diameter i, inner holes 1216 equally spaced with centers being 13.4 mm from the center of center hole 1218.

Center hole 1218 has a diameter j of approximately 5/8 inches (15.9 mm) and is threaded to accept a lower portion 1146 of piston shaft 1114.

Prior to use, the stainless steel screens (not shown) of the piston head 1118 and cylinder 1120 should be inspected for clogging, holes or over-stretching and replaced when necessary. A urine permeability measurement apparatus with damaged screen can deliver erroneous UPM results, and must not be used until the screen has been replaced.

A 5.00 cm mark 1156 is scribed on the cylinder 1120 at a height k of 5.00 cm ($\pm$0.05 cm) above the screen (not shown) attached to the bottom 1148 of the cylinder 1120. This marks the fluid level to be maintained during the analysis. Maintenance of correct and constant fluid level (hydrostatic pressure) is critical for measurement accuracy.

A constant hydrostatic head reservoir 1014 is used to deliver salt solution 1032 to the cylinder 1120 and to maintain the level of salt solution 1032 at a height k of 5.00 cm above the screen (not shown) attached to the bottom 1148 of the cylinder 1120. The bottom 1034 of the air-intake tube 1010 is positioned so as to maintain the salt solution 1032 level in the cylinder 1120 at the required 5.00 cm height k during the measurement, i.e., bottom 1034 of the air tube 1010 is in approximately same plane 1038 as the 5.00 cm mark 1156 on the cylinder 1120 as it sits on the cover plate 1047 and supporting ring 1040 (with circular inner opening of not less than 64 mm diameter) above the receiving vessel 1024.

The cover plate 1047 and supporting ring 1040 are parts as used in the equipment used for the method "K(t) Test Method (Dynamic Effective Permeability and Uptake Kinetics Measurement Test method)" as described herein and is called "Zeitabhängiger Durchlässigkeitsprüfstand" or "Time Dependent Permeability Tester", Equipment No. 03-080578 and is commercially available at BRAUN GmbH, Frankfurter Str. 145, 61476 Kronberg, Germany. Upon request, detailed technical drawings are also available.

Proper height alignment of the air-intake tube 1010 and the 5.00 cm mark 1156 on the cylinder 1120 is critical to the analysis. A suitable reservoir 1014 consists of a jar 1030 containing: a horizontally oriented L-shaped delivery tube 1018 connected to a flexible tube 1045 (e.g. Tygon tube, capable to connect nozzle and reservoir outlet) and to a Tygon tube nozzle 1044 (inner diameter at least 6.0 mm, length appr. 5.0 cm) for fluid delivery, a vertically oriented open-ended tube 1010 for admitting air at a fixed height within the constant hydrostatic head reservoir 1014, and a stoppered vent 1012 for re-filling the constant hydrostatic head reservoir 1014. Tube 1010 has an internal diameter of approximately 12 mm, but not less than 10.5 mm. The delivery tube 1018, positioned near the bottom 1042 of the constant hydrostatic head reservoir 1014, contains a stopcock 1020 for starting/stopping the delivery of salt solution 1032. The outlet 1044 of the delivery flexible tube 1045 is dimensioned (e.g. outer diameter 10 mm) to be inserted through the second lid opening 1136 in the cylinder lid 1116, with its end positioned below the surface of the salt solution 1032 in the cylinder 1120 (after the 5.00 cm height of the salt solution 1032 is attained in the cylinder 1120). The air-intake tube 1010 is held in place with an o-ring collar 1049. The constant hydrostatic head reservoir 1014 can be positioned on a laboratory reck 1016 at a suitable height relative to that of the cylinder 1120. The components of the constant hydrostatic head reservoir 1014 are sized so as to rapidly fill the cylinder 1120 to the required height (i.e., hydrostatic head) and maintain this height for the duration of the measurement. The constant hydrostatic head reservoir 1014 must be capable of delivering salt solution 1032 at a flow rate of at least 2.6 g/sec for at least 10 minutes.

The piston/cylinder assembly 1028 is positioned on the supporting ring 1040 in the cover plate 1047 or suitable alternative rigid stand. The salt solution 1032 passing through the piston/cylinder assembly 1028 containing the swollen hydrogel layer 1318 is collected in a receiving vessel 1024, positioned below (but not in contact with) the piston/cylinder assembly 1028.

The receiving vessel 1024 is positioned on the balance 1026 which is accurate to at least 0.001 g. The digital output of the balance 1026 is connected to a computerized data acquisition system 1048.

Preparation of Reagents (not illustrated)

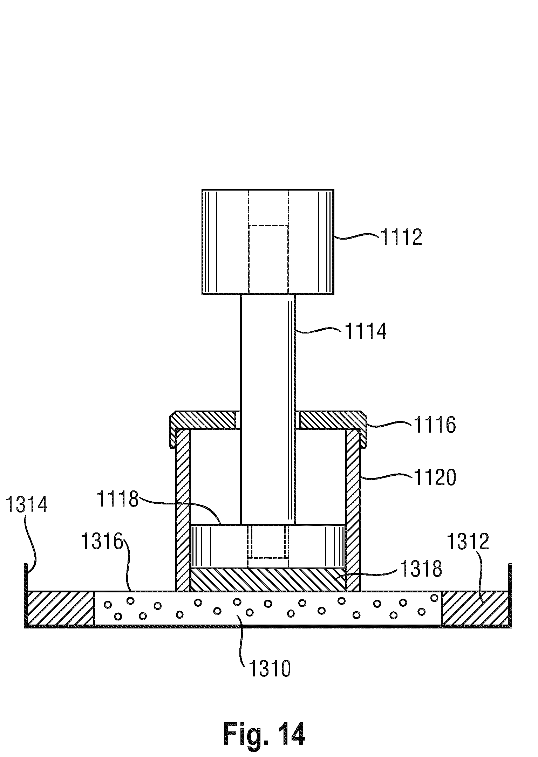

Jayco Synthetic Urine (JSU) 1312 (see Fig. 14) is used for a swelling phase (see UPM Procedure below) and 0.118 M Sodium Chloride (NaCl) Solution 1032 is used for a flow phase (see UPM Procedure below). The following preparations are referred to a standard 1 liter volume. For preparation of volumes other than 1 liter, all quantities are scaled accordingly.

JSU: A 1L volumetric flask is filled with distilled water to 80% of its volume, and a magnetic stir bar is placed in the flask. Separately, using a weighing paper or beaker the following amounts of dry ingredients are weighed to within $\pm$ 0.01 g

using an analytical balance and are added quantitatively to the volumetric flask in the same order as listed below. The solution is stirred on a suitable stir plate until all the solids are dissolved, the stir bar is removed, and the solution diluted to 1L volume with distilled water. A stir bar is again inserted, and the solution stirred on a stirring plate for a few minutes more.

Quantities of salts to make 1 liter of Jayco Synthetic Urine:

Potassium Chloride (KCl) 2.00 g
Sodium Sulfate (Na2SO4) 2.00 g
Ammonium dihydrogen phosphate (NH4H2PO4) 0.85 g
Ammonium phosphate, dibasic ((NH4)2HPO4) 0.15 g
Calcium chloride (CaCl2) 0.19 g - [or hydrated calcium chloride (CaCl2•2H2O) 0.25 g]
Magnesium chloride (MgCl2) 0.23 g - [or hydrated magnesium chloride (MgCl2•6H2O) 0.50 g]

To make the preparation faster, potassium chloride, sodium sulfate, ammonium dihydrogen phosphate, ammonium phosphate (dibasic) and magnesium chloride (or hydrated magnesium chloride) are combined and dissolved in the 80% of distilled water in the 1L volumetric flask. Calcium chloride (or hydrated calcium chloride) is dissolved separately in approximately 50 ml distilled water (e.g. in a glass beaker) and the calcium chloride solution is transferred to the 1L volumetric flask after the other salts are completely dissolved therein. Afterwards, distilled water is added to 1L (1000 ml ± 0.4 ml) and the solution is stirred for a few minutes more. Jayco synthetic urine may be stored in a clean plastic container for 10 days. The solution should not be used if it becomes cloudy.

0.118 M Sodium Chloride (NaCl) Solution: 0.118 M Sodium Chloride is used as salt solution 1032. Using a weighing paper or beaker 6.90 g (± 0.01 g) of sodium chloride is weighed and quantitatively transferred into a 1L volumetric flask (1000 ml ± 0.4 ml); and the flask is filled to volume with distilled water. A stir bar is added and the solution is mixed on a stirring plate until all the solids are dissolved.

The conductivity of the prepared Jayco solution must be in the range of appr. 7.48-7.72 mS/cm and of the prepared 0.118 M Sodium Chloride (NaCl) Solution in the range of appr. 12.34-12.66 mS/cm (e.g. measured via COND 70 INSTRUMENT without CELL, #50010522, equipped with Cell VPT51-01 C=0.1 from xs instruments or via LF 320 / Set, #300243 equipped with TetraCon 325 from WTW or COND 330i , #02420059 equipped with TetraCon 325 from WTW). The surface tension of each of the solutions must be in the range of 71-75 mN/m (e.g. measured via tensiometer K100 from Kruess with Pt plate).

Test Preparation

Using a solid reference cylinder weight (not shown) (50 mm diameter; 128 mm height), a caliper gauge (not shown) (measurement range 25 mm, accurate to 0.01 mm, piston pressure max. 50 g; e.g. Mitutoyo Digimatic Height Gage) is set to read zero. This operation is conveniently performed on a smooth and level bench (not shown) of at least approximately 11.5 cm x 15 cm. The piston/cylinder assembly 1028 without superabsorbent polymer particles is positioned under the caliper gauge (not shown) and a reading, L1, is recorded to the nearest 0.01 mm.

The constant hydrostatic head reservoir 1014 is filled with salt solution 1032. The bottom 1034 of the air-intake tube 1010 is positioned so as to maintain the top part (not shown) of the liquid meniscus (not shown) in the cylinder 1120 at the 5.00 cm mark 1156 during the measurement. Proper height alignment of the air-intake tube 1010 at the 5.00 cm mark 1156 on the cylinder 1120 is critical to the analysis.

The receiving vessel 1024 is placed on the balance 1026 and the digital output of the balance 1026 is connected to a computerized data acquisition system 1048. The cover plate 1047 with the supporting ring 1040 is positioned above the receiving vessel 1024.

UPM Procedure

1.5 g (± 0.05g) of superabsorbent polymer particles is weighed onto a suitable weighing paper or weighing aid using an analytical balance. The moisture content of the superabsorbent polymer particles is measured according to the Edana Moisture Content Test Method NWSP 230.0.R2 (15) or via a Moisture Analyzer (HX204 from Mettler Toledo, drying temperature 130°C, starting superabsorber weight 3.0 g (± 0.5 g), stop criterion 1 mg/ 140 s). If the moisture content of the superabsorbent polymer particles is greater than 3wt%, then the superabsorbent polymer particles are dried to a moisture level of < 3wt%, e.g. in an oven at 105°C for 3 h or e.g. at 120°C for 2 h.

The empty cylinder 1120 is placed on a level benchtop 1046 (not shown) and the superabsorbent polymer particles are quantitatively transferred into the cylinder 1120. The superabsorbent polymer particles are evenly dispersed on the screen (not shown) attached to the bottom 1148 of the cylinder 1120 while rotating the cylinder 1120, e.g. aided by a (manual or electrical) turn table (e.g. petriturn-E or petriturn-M from Schuett). It is important to have an even distribution of particles on the screen (not shown) attached to the bottom 1148 of the cylinder 1120 to obtain the highest precision result.

After the superabsorbent polymer particles have been evenly distributed on the screen (not shown) attached to the bottom 1148 of the cylinder 1120 particles must not adhere to the inner cylinder walls 1150. The piston shaft 1114 is inserted through the first lid opening 1134, with the lip 1154 of the lid 1116 facing towards the piston head 1118. The piston head 1118 is carefully inserted into the cylinder 1120 to a depth of a few centimeters. The lid 1116 is then placed onto the upper rim 1144 of the cylinder 1120 while taking care to keep the piston head 1118 away from the superabsorbent polymer particles. The weight 1112 is positioned on the upper portion 1128 of the piston shaft 1114 so that it rests on the shoulder 1124 such that the first and second linear index marks are aligned. The lid 1116 and piston shaft 1126 are then carefully rotated so as to align the third, fourth, fifth, and sixth linear index marks are then aligned with the first and the second linear index marks. The piston head 1118 (via the piston shaft 1114) is then gently lowered to rest on the dry superabsorbent polymer particles. Proper seating of the lid 1116 prevents binding and assures an even distribution of the weight on the hydrogel layer 1318.

Swelling Phase:

A fritted disc of at least 8 cm diameter (e.g. 8-9 cm diameter) and at least 5.0 mm thickness (e.g. 5-7 mm thickness) with porosity "coarse" or "extra coarse" ( e.g. Chemglass Inc. # CG 201-51, coarse porosity; or e.g. Robu 1680 with porosity 0) 1310 is placed in a wide flat-bottomed Petri dish 1314 and JSU 1312 is added by pouring JSU 1312 onto the center of the fritted disc 1310 until JSU 1312 reaches the top surface 1316 of the fritted disc 1310. The JSU height must not exceed the height of the fritted disc 1310. It is important to avoid any air or gas bubbles entrapped in or underneath the fritted disc 1310.

The entire piston/cylinder assembly 1028 is lifted and placed on the fritted disc 1310 in the Petri dish 1314. JSU 1312 from the Petri dish 1314 passes through the fritted disc 1310 and is absorbed by the superabsorbent polymer particles (not shown) to form a hydrogel layer 1318. The JSU 1312 available in the Petri dish 1314 should be enough for all the swelling phase. If needed, more JSU 1312 may be added to the Petri dish 1314 during the hydration period to keep the JSU 1312 level at the top surface 1316 of the fritted disc 1310. After a period of 60 minutes, the piston/cylinder assembly 1028 is removed from the fritted disc 1310, taking care to ensure the hydrogel layer 1318 does not lose JSU 1312 or take in air during this procedure. The piston/cylinder assembly 1028 is placed under the caliper gauge (not shown) and a reading, L2, is recorded to the nearest 0.01 mm. If the reading changes with time, only the initial value is recorded. The thickness of the hydrogel layer 1318, L0 is determined from L2 - L1 to the nearest 0.1 mm.

The piston/cylinder assembly 1028 is transferred to the supporting ring 1040 in the cover plate 1047. The constant hydrostatic head reservoir 1014 is positioned such that the delivery tube nozzle 1044 is placed through the second lid opening 1136. The measurement is initiated in the following sequence:

a) The stopcock 1020 of the constant hydrostatic head reservoir 1014 is opened to permit the salt solution 1032 to reach the 5.00 cm mark 1156 on the cylinder 1120. This salt solution 1032 level should be obtained within 10 seconds of opening the stopcock 1020.
b) Once 5.00 cm of salt solution 1032 is attained, the data collection program is initiated.

With the aid of a computer 1048 attached to the balance 1026, the quantity g (in g to accuracy of 0.001 g) of salt solution 1032 passing through the hydrogel layer 1318 is recorded at intervals of 20 seconds for a time period of 10 minutes. At the end of 10 minutes, the stopcock 1020 on the constant hydrostatic head reservoir 1014 is closed.

The data from 60 seconds to the end of the experiment are used in the UPM calculation. The data collected prior to 60 seconds are not included in the calculation.

For each time period of 20 seconds (time $t_{(i-1)}$ to $t_i$) after the initial 60 seconds of the experiment, the respective flow rate $Fs_{(t)}$ (in g/s) and the respective mid-point of the time $t_{(1/2)t}$ (in s) is calculated according to the following formulas:

$$Fs_{(t)} = \frac{(g_{(i-1)} - g_{(i)})}{(t_{(i-1)} - t_{(i)})}$$

and

$$t_{(1/2)_t} = \frac{(t_{(i-1)} + t_{(i)})}{2} \quad \text{(II)}$$

The flow rate $Fs_{(t)}$ of each time interval ($t_{(i-1)}$ to $t_i$) is plotted versus the mid-point of the time $t_{(1/2)t}$ of the time interval ($t_{(i-1)}$ to $t_i$). The intercept is calculated as Fs(t=0).

Calculation of the Intercept:

The intercept is calculated via a best-fit regression line, e.g. as following: the equation for the intercept of the regression line, a, is:

$$a = y_{AVG} - b \cdot x_{AVG} \qquad \text{(III)}$$

where the slope, b, is calculated as:

$$b = \frac{\sum(x - x_{AVG}) \cdot (y - y_{AVG})}{\sum(x - x_{AVG})^2} \qquad \text{(IV)}$$

and where $x_{AVG}$ and $y_{AVG}$ are the sample means AVERAGE of the known_x's and AVERAGE of the known_y's, respectively.

Calculation of Urine Permeability Measurement Q:

The intercept Fs(t=0) is used to calculate Q according to the following formula:

$$Q = \frac{F_s(t=0) \cdot L_0}{\rho \cdot A \cdot \Delta P} \qquad \text{(V)}$$

where the flow rate Fs(t=0) is given in g/s, $L_0$ is the initial thickness of the hydrogel layer 1318 in cm, $\rho$ is the density of the salt solution 1032 in $g/cm^3$ (e.g. 1.003 $g/cm^3$ at room temperature). A (from the equation above) is the area of the hydrogel layer 1318 in $cm^2$ (e.g. 28.27 $cm^2$), $\Delta P$ is the hydrostatic pressure in $dyne/cm^2$ (e.g. 4920 $dyne/cm^2$), and the Urine Permeability Measurement, Q, is in units of $cm^3 sec/g$. The average of three determinations should be reported.

| Variable | Description | Unit |
|---|---|---|
| $g_i$ | Mass of salt solution 1032 flown through the swollen gel layer (recorded by the balance) at the time $t_i$ (accuracy 0.001 g) | g |
| $t_i$ | Time point (every 20 s) | s |
| $t_{(1/2)t}$ | Mid-point of time for the respective time interval $t_{i-1}$ to $t_i$ | s |
| $Fs_t$ | Flow Rate at the time interval $t_{i-1}$ to $t_i$ | g/s |
| Fs (t=0) | Intercept flow rate at t = 0 s from the plot of the flow rate Fs(t) vs. the mid-point of time $t_{(1/2)t}$. | g/s |
| $L_0$ | Thickness of the swollen gel layer (swollen with JSU 1312) before the salt solution 1032 flows through the gel layer. | Cm |
| $\rho$ | Density of the salt solution 1032 (1.003 $g/cm^3$) | $g/cm^3$ |
| A | Area of the swollen gel layer (28.27 $cm^2$) | $cm^2$ |
| $\Delta P$ | Hydrostatic pressure across the gel layer (4920 $dyne/cm^2$) | $dyne/cm^2$ |
| Q | Urine Permeability Measurement | $cm^3 * sec/g$ |

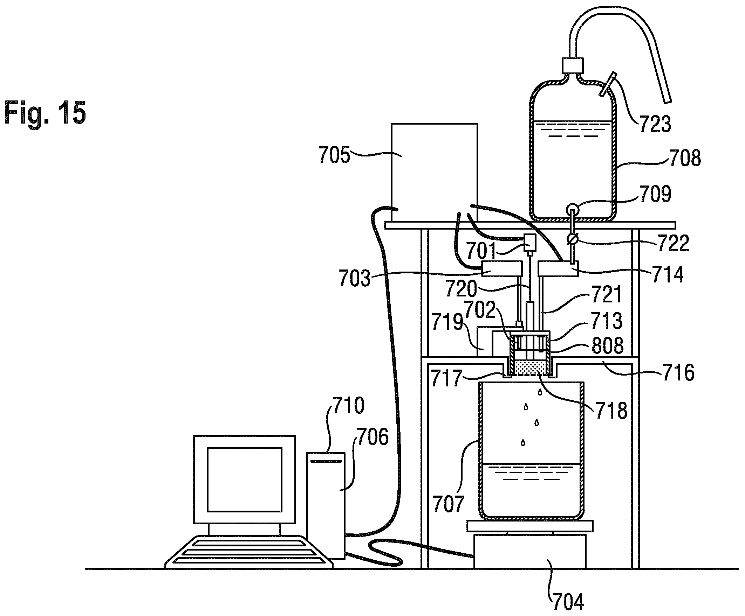

## SAP K(t) Test Method (Figs. 15-17)

This method determines the time dependent effective permeability (SAP K(t)) and the uptake kinetics of a gel layer formed from hydrogel-forming superabsorbent polymer particles or of an absorbent structure containing such particles under a confining pressure. The objective of this method is to assess the ability of the gel layer formed from hydrogel-forming superabsorbent polymer particles or the absorbent structure containing them to acquire and distribute body fluids when the polymer is present at high concentrations in an absorbent article and exposed to mechanical pressures as they typically occur during use of the absorbent article. Darcy's law and steady-state flow methods are used to calculate effective permeability (see below). (See also for example, "Absorbency," ed. By P.K. Chatterjee, Elsevier, 1982, Pages 42-43 and "Chemical Engineering Vol. II, Third Edition, J.M. Coulson and J.F. Richardson, Pergamon Press, 1978, Pages 122-127.)

In contrast to previously published methods, the sample is not preswollen therefore the hydrogel is not formed by preswelling hydrogel-forming superabsorbent polymer particles in synthetic urine, but the measurement is started with a dry structure. The equipment used for this method is called 'Zeitabhängiger Durchlässigkeitsprüfstand' or 'Time Dependent Permeability Tester', Equipment No. 03-080578 and is commercially available at BRAUN GmbH, Frankfurter Str. 145, 61476 Kronberg, Germany and is described below. Upon motivated request, operating instructions, wiring diagrams and detailed technical drawings are also available.

**Dynamic Effective Permeability and Uptake Kinetic Measurement System**

Fig. 15 shows the dynamic effective permeability and uptake kinetic measurement system, called 'Time Dependent Permeability Tester' herein. The equipment consists of the following main parts:

- M11 Digital Laser Sensor for caliper measurement 701 (MEL Mikroelektronik GmbH, 85386 Eching, Germany) or equivalent (e.g. Keyence II-S100 Laser Height Sensor).
- Fiber for Liquid Level Detection 702 (FU95, Keyence Corp., Japan)
- Digital Fiber Sensor 703 (FS-N10, Keyence Corp., Japan)
- Precision Balance 704 (XP6002MDR, Mettler Toledo AG, 8606 Greifensee, Switzerland)
- Power Unit Logo!Power (C98130-A7560-A1-5-7519, Siemens AG)
- Labview Software License 706 (National Instruments, Austin, Tx, USA)
- Receiving Vessel 707 (5L Glass Beaker, Roth)
- Reservoir 708 (5L Glass bottle, VWR) with joint 709 and open-end tube for air admittance 723
- Operating unit and console 705(Conrad Electronics)
- Computerized data acquisition system 710
- A piston/cylinder assembly 713 as described herein
- A controlled valve 714 (Bürkert)

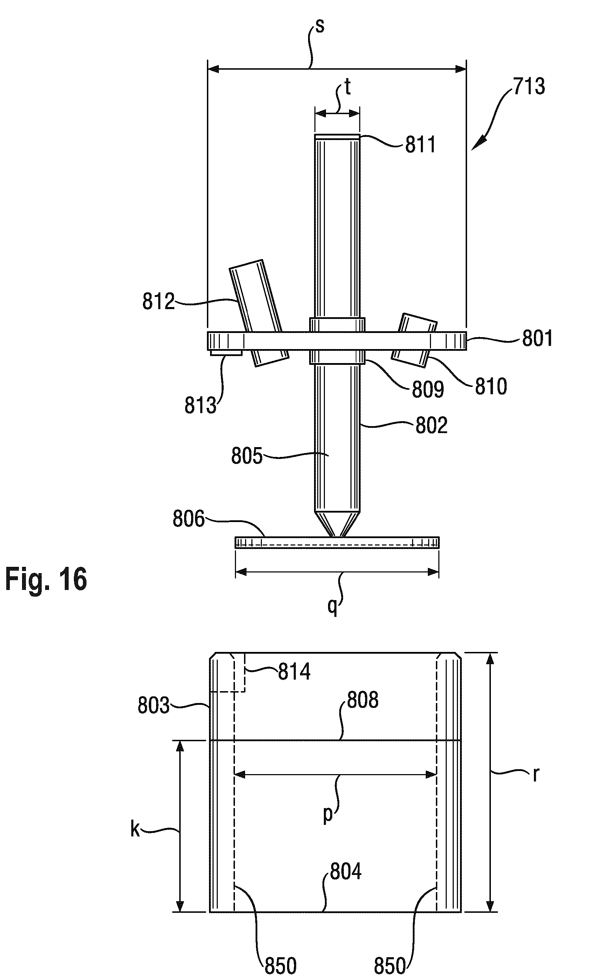

Fig. 16 shows the piston/cylinder assembly 713 comprising piston guiding lid 801, piston 802 and cylinder 803. The cylinder 803 is made of transparent polycarbonate (e.g., Lexan®) and has an inner diameter p of 6.00 cm (area=28.27 cm$^2$). The inner cylinder walls 850 are smooth; the height of the cylinder r is about 7.50 cm. The bottom 804 of the cylinder 803 is faced with a US. Standard 400 mesh stainless-steel screen cloth (not shown) (e.g. from Weisse and Eschrich) that is bi-axially stretched to tautness prior to attachment to the bottom 804 of the cylinder 803. The piston 802 is composed of a stainless steel piston body 805 and a stainless steel head 806. The piston head 806 diameter q is slightly less than 6 cm so as to slide freely into the cylinder 803 without leaving any gap for the hydrogel-forming particle to pass through. The piston body 805 is firmly attached perpendicularly at the center of the piston head 806. The piston body diameter t is about 2.2 cm. The piston body 805 is then inserted into a piston guiding lid 801. The guiding lid 801 has a POM (Polyoxymethylene) ring 809 with a diameter allowing a free sliding of the piston 802 yet keeping the piston body 805 perfectly vertical and parallel to the cylinder walls 850 once the piston 802 with the guiding lid 801 are positioned on top of the cylinder 803. The top view of the piston head 806 is shown in Fig. 16. The piston head 806 is meant to apply the pressure homogeneously to the sample 718. It is also highly permeable to the hydrophilic liquid so as to not limit the liquid flow during measurement. The piston head 806 is composed of a US. Standard 400 mesh stainless steel screen cloth 903 (e.g. from Weisse and Eschrich) that is bi-axially stretched to tautness and secured at the piston head stainless steel outer ring 901. The entire bottom surface of the piston is flat. Structural integrity and resistance to bending of the mesh screen is then ensured by the stainless steel radial spokes 902. The height of the piston body 805 is selected such that the weight of the piston 802 composed of the piston body 805 and the piston head 806 is 596 g ($\pm$6g), this corresponds to 0.30 psi over the area of the cylinder 803.

The piston guiding lid 801 is a flat circle of stainless steel with a diameter s of about 7.5 cm held perpendicular to the piston body 805 by the POM ring 809 in its center. There are two inlets in the guiding lid (810 and 812).

The first inlet 812, allows the Fiber for Liquid Level Detection 702 to be positioned exactly 5 cm above the top surface of the screen (not shown) attached to the bottom (804) of the cylinder 803 once the piston 802 is assembled with the cylinder 803 for the measurement.

The second inlet 810 allows connecting a liquid tube 721 providing the liquid to the experiment. To make sure that the assembly of the piston 802 with the cylinder 803 is done consistently a slit 814 is made on the cylinder 803 matching a position marker 813 in the guiding lid 801. In this way the rotation angle of the cylinder and the guiding lid is always the same.

Prior to every use, the stainless steel screen cloth 903 of the piston head 806 and cylinder 803 should be inspected for clogging, holes or over-stretching and replaced when necessary. A K(t) apparatus with damaged screen can deliver erroneous K(t) and uptake kinetic results, and must not be used until the screen has been replaced.

A 5 cm mark 808 is scribed on the cylinder at a height k of 5.00 cm ($\pm$0.02 cm) above the top surface of the screen attached to the bottom 804 of the cylinder 803. This marks the fluid level to be maintained during the analysis. The Fiber for Liquid Level Detection 702 is positioned exactly at the 5 cm mark 808. Maintenance of correct and constant fluid level (hydrostatic pressure) is critical for measurement accuracy

A reservoir 708 connected via tubing to the piston/cylinder assembly 713 holding the sample and a controller valve 714 are used to deliver salt solution to the cylinder 803 and to maintain the level of salt solution at a height k of 5.00 cm above the top surface of screen attached to the bottom of the cylinder 804. The valve 714, the Fiber for Liquid Level Detection 702 and the Digital Fiber Sensor 703 are connected to the computerized acquisition system 710 through the operating unit 705. This allows the Dynamic Effective Permeability and Uptake Kinetic Measurement System to use the information from the Fiber for Liquid Level Detection 702 and the Digital Fiber Sensor 703 to control the valve 714 and ultimately maintain the level of the liquid at the 5 cm mark 808.

The reservoir 708 is placed above the piston/cylinder assembly 713 in such a manner as to allow a 5 cm hydrohead to be formed within 15 seconds of initiating the test, and to be maintained in the cylinder throughout the test procedure. The piston/cylinder assembly 713 is positioned on the support ring 717 of the cover plate 716 and the first inlet 812 is held in place with the docking support 719. This allows only one position of the guiding lid 801. Furthermore, due to the position marker 813, there is also only one position for the cylinder 803. The screen attached to the bottom of the cylinder 804 must be perfectly level and horizontal. The supporting ring 717 needs to have an internal diameter small enough, so to firmly support cylinder 803 but larger than 6.0 cm so to lay outside of the internal diameter of the cylinder once the cylinder is positioned on the supporting ring 717. This is important so to avoid any interference of the supporting ring 717 with the liquid flow.

The salt solution, applied to the sample 718 with a constant hydrohead of 5 cm can now freely flow from the piston/cylinder assembly 713 into a receiving vessel 707 positioned on the balance 704 which is accurate within $\pm$ 0.01 g. The digital output of the balance is connected to a computerized data acquisition system.

The thickness (caliper) of the sample is constantly measured with a Digital Laser Sensor for caliper measurement 701. The laser beam 720 of the digital laser sensor 701 is directed at the center of the POM cover plate 811 of the piston body. The accurate positioning of all the parts of the piston/cylinder assembly 713 allows the piston body 805 to be perfectly parallel to the laser beam 720 and as a result an accurate measure of the thickness is obtained.

Test Preparation: the reservoir 708 is filled with test solution. The test solution is an aqueous solution containing 9.00 grams of sodium chloride and 1.00 grams of surfactant per liter of solution. The preparation of the test solution is described below. The receiving vessel 707 is placed on the balance 704 which is connected to a computerized data acquisition system 710. Before the start of the measurement the balance is reset to zero.

Preparation of test liquid: Chemicals needed:

- Sodium Chloride (CAS#7647-14-5, eg: Merck, cat# 1.06404.1000)
- Linear $C_{12}$-$C_{14}$ alcohol ethoxylate (CAS#68439-50-9, eg. Lorodac ®, Sasol, Italy)
- Deionized $H_2O$

Ten liters of a solution containing 9.00 grams per litre of NaCl and 1.00 grams per liter linear C12-C14 alcohol ethoxalate in distilled water is prepared and equilibrated at 23°C $\pm$ 1°C for 1 hour. The surface tension is measured on 3 individual aliquots and should be 30+- 2mN/m. If the surface tension of the solution is different from 28 $\pm$ 0.5 mN/m, the solution is discarded and a new test solution is prepared. The test solution has to be used within 3 months from its preparation and is considered expired afterwards.

SAP K(t) Sample preparation: The superabsorbent polymer particles are dried e.g. in a circulation oven at atmospheric pressure at 120°C for 2 hours to remove excess moisture before measurement. The particles can then be stored in a tightly sealed airtight container at 23 $\pm$ 2°C until further use or directly used for the measurement.

2.0 g ($\pm$0.02 g) of superabsorbent polymer particles are weighed onto a suitable weighing paper using an analytical balance and transferred to the cylinder 803 with the particles distributed evenly on the screen (not shown) attached to the bottom 804 of the cylinder 803. This is done via sprinkling the superabsorbent polymer, while at the same time turning the cylinder clockwise (e.g. on a circular turning table schuett petriturn-M available at Schuett-biotec GmbH, Rudolf-Wissell-Str. 13 D-37079 Göttingen Germany). An even distribution of the superabsorbent polymer particles is critical for the accuracy of the measurement.

SAP K(t) Procedure: The measurement is carried out at controlled lab conditions: 23°C $\pm$1°C/45% RH $\pm$10%. Control of lab condition can be done for example via Opus 20 E from G. Lufft Mess- und Regeltechnik GmbH. The empty piston/cylinder assembly 713 is mounted in the circular opening in the cover plate 716 and is supported around its lower perimeter by the supporting ring 717. The piston/cylinder assembly 713 is held in place with the docking support 719 with the cylinder 803 and piston 802 aligned at the proper angle. The reference caliper reading ($r_r$) is measured by Digital Laser sensor. After this, the empty piston/cylinder assembly 713 is removed from the cover plate 716 and supporting ring 717 and the piston 802 is removed from the cylinder 803.

The sample 718 is positioned (absorbent structure) or sprinkled (superabsorbent polymer particles) on the cylinder screen as explained above. After this, the piston 802 assembled with the guiding lid 801 is carefully set into the cylinder 803 by matching the position marker 813 of the guiding lid 801 with the slit 814 made in the cylinder 803

The piston/cylinder assembly is held in place with the docking support 719 with the cylinder and piston aligned at the proper angle.

This can be only done in one way. The liquid tube 721 connected to the reservoir 708 and the Digital Fiber Sensor 703 are inserted into the piston/cylinder assembly 713 via the two inlets 810 and 812 in the guiding lid 801.

The computerized data acquisition system 710 is connected to the balance 704 and to the digital laser sensor for caliper measurement 701. Fluid flow from the reservoir 708 to the cylinder 803 is initiated by the computer program by opening valve 714. The cylinder is filled until the 5 cm mark 808 is reached in 5 to 15 seconds, after which the computer program regulates the flow rate to maintain a constant 5 cm hydrohead. The quantity of solution passing through the sample 718 is measured by the balance 704 and the caliper increase is measured by the laser caliper gauge. Data acquisition is started when the fluid flow is initiated specifically when the valve 714 is opened for the first time, and continues for 21 minutes or until the reservoir runs dry so that the 5 cm hyrdrohead is no longer maintained. The duration of one measurement is 21 min, laser caliper and balance readings are recorded regularly with an interval that may vary according to the measurement scope from 2 to 10sec, with typically 10sec interval, and 3 replicates are measured.

After 21 min, the measurement of the 1st replicate is successfully completed and the controlled valve 714 closes automatically. The piston/cylinder assembly 713 is removed and the measurements of the 2nd and 3rd replicates are done accordingly, always following the same procedure. At the end of the measurement of the 3rd replicate, the controlled valve 714 stops the flow of liquid and stopcock 722 of the reservoir 708 is closed. The collected raw data is stored in the form of a simple data table, which then can be imported easily to a program for further analysis e.g. Excel 2003, SP3.

In the data table the following relevant information is reported for each reading:

- Time from the beginning of the experiment
- Weight of the liquid collected by the receiving vessel 707 on the balance 704
- Caliper of the sample 718

The data from 100 seconds to the end of the experiment are used in the K(t) and uptake kinetics calculation. The data collected in the first 100 seconds are not included in the calculation. The effective permeability K(t) and the uptake kinetics of the absorbent structure are then determined using the equation sets below.

Used equations: The table below describes the notation used in the equations.

| | |
|---|---|
| A | x-section of the absorbent structure sample which corresponds to the cylinder inner radius: 28.27 cm$^2$ |
| h | height of water column, 5.0 cm |
| $\Delta p$ | driving pressure applied by the 5.00 cm hydrohead (h) : 4929.31 g / (cm s$^2$) |
| G | gravity constant: 981 cm/ s$^2$ |
| $\eta$ | Temperature dependent effective viscosity of the liquid in g/(cm s) |
| T | Temperature in °C |
| $\rho$ | density of the liquid: 1.0053 g/cm$^3$ |
| $\rho_s{}^A$ | Apparent sample density of the porous medium or powder in g/cm$^3$ |
| $\rho_s$ | Average density of the solid part of the dry sample in g/cm$^3$ |
| $P_{sk}$ | Density of the component k of the dry sample in g/cm$^3$ |
| m | dry mass of the sample in g: 2.00 g if measuring superabsorbent particles |
| $m_k$ | Mass of the component k of the dry sample in g |
| $V_s$ | Dry sample volume in cm$^3$ |
| $t_i$ | time at step i of N discrete points in s |
| $d_i$ | caliper of the absorbent structure sample at time $t_i$ in cm |
| $r_i$ | reading of caliper instrument at time $t_i$ in cm |
| $r_r$ | reference reading of caliper instrument (reading of the piston/cylinder assembly without sample) in cm |
| $m_{out\,i}$ | balance reading at time $t_i$; mass of the liquid that left the sample at time $t_i$ in g |

(continued)

| U(t$_i$) | Sample uptake at time t$_i$ in g |
|---|---|
| T20 | time required to reach an uptake of 20 g/g, starting at 0 s (t$_0$) in s |
| T15 | time required to reach an uptake of 15 g/g, starting at 0 s (t$_0$) in s |
| U20 | Sample uptake after 20 minutes in g/g |
| T80% | Time required to reach an uptake of 80% of U20 starting at 0 s (t$_0$) in s |
| K20 | Sample permeability at 20 minutes in cm$^2$ |
| Kmin | the minimum value of the permeability during the experiment in m$^2$ |
| Kmin/K20 | the ratio of Kmin and K20 |

The driving pressure is calculated from the hydro head as follows:

$$\Delta p = h \cdot G \cdot \rho = 4929.31 \text{ g/(cm} \cdot s^2)$$

The caliper at each time t$_i$ is calculated as the difference of the caliper sensor reading at time t$_i$ and the reference reading without sample:

$$d_i = r_i - r_r \ [\text{cm}]$$

For superabsorbent particles samples the caliper of the sample at time t$_i$=0 (d$_0$) is used to evaluate the quality of the particle sprinkling.

An apparent sample density inside the cylinder can be in fact calculated as:

$$\rho_s^A = \frac{m}{d_0 \cdot A} \quad [\text{g/cm}^3]$$

If this apparent density inside the cylinder differs from the apparent density of the powder by more than ± 40% the measurement has to be considered invalid and eliminated.

The apparent density can be measured according EDANA method NWSP 251.0.R2(19) PSP - Gravimetric Determination of Flow Rate and Bulk Density.

The rate of change with time of the balance reading at time t$_i$ is calculated as follows:

$$\frac{dm_{out}(t_i)}{dt} = \frac{m_{out\ i+1} - m_{out\ i-1}}{t_{i+1} - t_{i-1}} \ [\text{g/sec}]$$

The rate of change with time of the caliper reading at time t$_i$ is calculated as follows:

$$\frac{dd(t_i)}{dt} = \frac{d_{i+1} - d_{i-1}}{t_{i+1} - t_{i-1}} \ [\text{cm/sec}]$$

The uptake Kinetics is calculated as follows:

$$U(t_i) = \frac{(A \cdot d_i - V_S) \cdot \rho}{m} \quad [\text{g/g}]$$

By dry sample volume (V$_s$) is intended the skeletal volume of the sample therefore V$_s$ is the actual volume occupied by the solid material in the dry sample excluding pores and interstitials that might be present.

V$_s$ can be calculated or measured by different methods known by the skilled person for example, knowing the exact composition and the skeletal density of the components it can be determined as follows:

$$Vs = \sum_k V_k = \sum_k \frac{m_k}{\rho_{S\,k}} \quad [\text{cm}^3]$$

Alternatively for an unknown material composition $V_s$ can be easily calculated as follow:

$$V_S = \frac{m}{\rho_S} \quad [\text{cm}^3]$$

The average density $\rho_s$ can be determined by pycnometry with a suitable non-swelling liquid of known density (ethanol in the present case). This technique cannot be performed on the same samples subsequently used for the K(t) measure therefore a suitable additional representative set of samples should be prepared for this experiment measurement. The average of at least three replicates is used to calculate the Average Specific Density. In case the Average Specific Density is unknown, a value of 1.50 g/cm$^3$ for an absorbent core and of 1.60 g/cm$^3$ for an SAP can be assumed as good approximation unless there is evidence for significant deviation from these values (e.g. in case of extremely porous SAP particles or in case of absorbent cores with extremely low content of SAP particles).
From U(t) at the different time steps calculated as explained above, one can determine the uptake at any specific time by linear interpolation. For example one of the important outputs is the uptake at 20 minutes also called U20 (in g/g).
From U(t) at the different time steps one can also determine the time required to reach a certain uptake by linear interpolation. The time where the uptake of 20 g/g is first reached is called T20. Similarly the time to reach any other uptakes can be calculated accordingly, for example the time to reach 15 g/g (T15). Knowing U20 it is possible to determine from U(t) at the different time steps also the time to reach 80% of U20, this property is called T80%.
The Effective Permeability is calculated as follows from the rates of mass change and caliper change:

$$K(t_i) = \eta \, \frac{d_i}{\Delta p} \cdot \left( \frac{1}{\rho \cdot A} \cdot \frac{dm_{out}(t_i)}{dt} + \frac{dd(t_i)}{dt} \right) \quad [\text{cm}^2]$$

The effective viscosity of the liquid depends on the temperature and in the interval of the experiment (23°C $\pm$1°C) is calculated according the following empirical equation:

$$\eta = -2.36 \cdot 10^{-4} \cdot T + 1.479 \cdot 10^{-2} \ [\text{g/(cm s)}]$$

From K($t_i$) one can determine the effective permeability at a certain time by linear interpolation. For example one of the important outputs is the permeability at 20 minutes or K20 (cm$^2$). Similarly the Permeability at any other time can be calculated accordingly (e.g. K5 or K10).
Another parameter to be derived from the data is Kmin, which is the minimum K(t) value measured over the whole curve in the interval from $t_i$= 100s to $t_i$= 1200s. This value is useful to calculate Kmin/K20 which is the ratio between the minimum effective permeability and the permeability at 20 minutes. This parameter expresses the temporary gel blocking that might occur in some of the samples. If the value is close to 1 there is no temporary gel blocking if the value is close to 0 it is an indication that the material goes through a strong effective permeability drop when initially loaded with liquid.
The average values for T20, T80%, K20, U20 and Kmin/K20 are reported from 3 replicates according to the accuracy required as known by the skilled man.

**Absorbent Core K(t) Test Method**

The method described above can be easily adapted to measure the K(t) of the absorbent core directly without having to separate the SAP from the high loft layer. The equipment system and test liquid are the same as described above and illustrated in present the Figures for the SAP K(t) method described. This adapted test method has been first described in EP2,535,698A1 (Ehrnsperger et al., P&G) to measure the T15 and T20 of an absorbent core.

Method for Extracting An Absorbent Core From An Absorbent Article

The absorbent article is positioned on a flat surface. In case the product contains features that prevent it to lay flat (such as the cuff elastics) they are cut at suitable intervals to allow the product to lay flat. Any layers attached to the absorbent core such as topsheet or backsheet are removed from the absorbent core. To avoid damaging the core, these layers can be removed with the aid of Cold sprays with a cooling temperature from -50 to - 60 °C (such as "IT Icer" or "PRF 101 cold spray" available at Taerosol, Kangasala Finland) as shown for example in Fig. 15 of EP2535698. To avoid undue damage to the absorbent core, the layer of material to be removed from the absorbent core is pulled off the absorbent core in a 180° peel geometry while the adhesive material is cooled with the Cold spray. The spraying should last at least 1 seconds but no more than 5 seconds for each single portion of the layer of material. After removal of each material, the remaining part of the absorbent core is kept under 0.3 psi pressure until the temperature is back to the initial value (TAPPI lab condition).

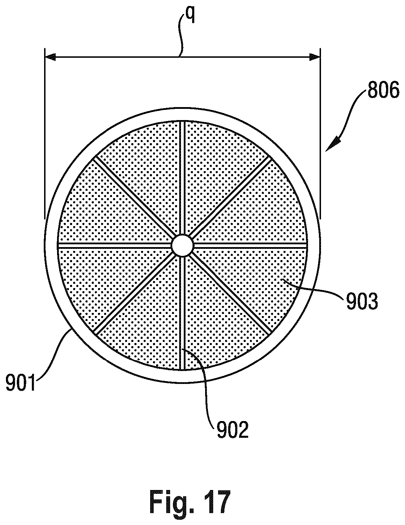

The upper layer and/or the lower layer of the absorbent core may be properly perforated to allow liquid flow through (as shown for example in Fig. 16 of EP2535698). The perforation is performed using a hot metal tip also called perforating tip which comprises a steel rod with a diameter H of $0.7 \pm 0.2$ mm. A standard paper clip, bend around a solder tip such as CT 60/621 available at ERSA GmbH, Wertheim, Germany can be used for the purpose. The perforating tip should be set at the temperature of $310 \pm 20$°C. The perforating tip is positioned in contact with the layers to be perforated for a short period of time with low pressure so to perforate the layers, for example by melting without affecting any of the other materials of the absorbent structure. The holes are created with the same procedure in a regular square perforation pattern with a hole edge to edge distance D of $1 \pm 0.2$ mm (as shown for example in Fig. 17 of EP2535698).

Each absorbent core is visually checked for integrity and discarded if damaged with the help of a back light. Examples of damages are for examples: cuts, holes, wrinkles which were not present before removing the absorbent structure from the absorbent article. The perforations in the layers done with the perforating tip, are not considered damages unless they affect other layers. The substantial migration of superabsorbent polymer particles and fibers within the absorbent structure is also considered damage.

The absorbent cores prepared as such are then cut to prepare a circular sample according to the K(t) Absorbent Core Sample preparation described below.

K(t) Absorbent Core Sample preparation

A circular portion of 6.00 cm diameter of the absorbent core is obtained from the center of the absorbent core. A suitable circular die and a hydraulic press cutter (like e.g. Electro-Hydraulic Alfa Cutter 240-10 available at Thwing-Albert instrument company, 14 W. Collings Ave.West Berlin, NJ 08091) can be used for this purpose.

The circular sample is carefully positioned flat on the screen (not shown) attached to the bottom of the cylinder occupying all the available surface on the screen. It is important to position the circular sample in a way that the side in direct contact with the screen is the one that in use is usually more distant from the liquid source so as to reproduce the common flow direction in use. For example for samples related to absorbent articles such as diapers, the side usually facing the wearer should be positioned on top while the side facing the garments should be positioned in contact with the screen at the bottom of the cylinder. A careful positioning of the sample is critical for the measurement's accuracy. In case the dimension of the absorbent core is small and a 6.0 cm diameter sample cannot be obtained from it, it is possible to join two absorbent cores of equal size so to obtain the minimum sample size necessary. The two samples need to be taken in the same position from two identical absorbent cores. The two absorbent cores should be joined through a straight edge and if necessary cut to obtain such a straight edge. The intent is that the joined edges recreate a flat homogeneous layer with no or only a minimal gap. This joint layer is then handled according to the standard sample preparation described above with the additional precaution to center the join line in the cutting die so to obtain two half circles of identical shape. It is important that both the half circles are carefully positioned inside the sample holder so to recreate a full circle and occupying the entire available surface on the screen with no or only a minimal gap. Both the halves have to be positioned with the side facing the screen as explained above. However, in most embodiments, the sample will consist of a unitary circular portion of the absorbent core.

The Absorbent Core K(t) method is conducted as indicated above, in particular a Core T15 and a Core K20 are determined in like manner. The Core Density at 0.3 psi can further be determined according to the formula:

$$\rho_{core} = \frac{m}{d_0 \cdot A}$$

where m is the mass of the absorbent core sample.

**Claims**

1. An absorbent core (28) for use in an absorbent article (20), the absorbent core extending in a transversal direction (x) and a longitudinal direction (y) and having a thickness in a vertical direction (z) perpendicular to the transversal direction and longitudinal direction, the absorbent core comprising:

EP 3 881 814 B1

- a liquid-permeable top layer (41);
- a bottom layer (42);
- a high loft central layer (43) having a density at a pressure of 4.14 kPa below 0.20 g/cm$^3$ between the top layer and the bottom layer, as measured according to the Thickness and Density Measurement Method as described herein;
- superabsorbent polymer particles between the top layer and the bottom layer, wherein the superabsorbent polymer particles are at least partially distributed within the central layer (43);

wherein the superabsorbent polymer particles have a time to reach an uptake of 20 g/g (SAP T20) of less than 220 s, as measured according to the SAP K(t) Test Method as described herein.

2. An absorbent core according to claim 1, wherein the absorbent core has a time to reach an uptake of 20 g/g (Core T20) of less than 550 s, as measured according to the Absorbent Core K(t) Test Method as described herein.

3. An absorbent core according to any of the preceding claims, wherein the central layer comprises or consists of synthetic fibers, in particular wherein the central layer is a carded nonwoven layer.

4. An absorbent core according to any of the preceding claims, wherein the concentration of the superabsorbent polymer particles in the absorbent core has a bi-modal or multimodal distribution in the z-direction of the absorbent core, as determined by the micro-CT Scan Method disclosed herein.

5. An absorbent core according to any of the preceding claims, wherein the superabsorbent particles are made of a cross-linked polyacrylate salt, and preferably have a Centrifuge Retention Capacity (CRC) in the range of from 10 g/g up to 35 g/g test as measured by EDANA method NWSP 241.0.R2 (19).

6. An absorbent core according to any of the preceding claims, wherein the superabsorbent polymer particles have a UPM of at least 10 × 10-7 cm3.s/g, preferably of at least 15 × 10-7 cm3.s/g, wherein the UPM is measured by the Urine Permeability Measurement Test described herein, and/or wherein the superabsorbent polymer particles have an Absorbency Against Pressure of 0.7 psi (AAP@0.7psi) of more than 22 g/g measured according to EDANA standard test NWSP 242.0 R2 (19).

7. An absorbent core according to any of the preceding claims, wherein the top layer and/or the bottom layer are each attached to the central layer by a layer of glue (71, 72), optionally wherein the layer of glue also immobilizes at least a portion of the SAP particles which are not distributed within the high loft central layer in the dry state.

8. An absorbent core according to any of the preceding claims, further comprising a wrapping layer (3), in particular a nonwoven wrapping layer, at least partially wrapping the top, bottom and central layers.

9. An absorbent core according to any of the preceding claims, wherein the absorbent core comprises at least 60% by weight of superabsorbent polymer particles, preferably at least 70% or at least 80% by weight of SAP, by total weight of the core.

10. An absorbent core according to any of the preceding claims, wherein the absorbent core comprises superabsorbent polymer particles at a basis weight of at least 200 gsm, or at least 300 gsm or from 300 gsm to 500 gsm.

11. An absorbent core according to any of the preceding claims, comprising a first central layer (431) and a second central layer (432) between the top layer and the bottom layer, wherein at least one of the first central layer and the second central layer is a central layer according to any of the preceding claims, and wherein the first central layer and the second central layer can be the same or different.

12. An absorbent core according to any of the preceding claims, wherein the absorbent core has a core density of less than 0.6 g/cm3, preferably from 0.2 g/cm$^3$ to less than 0.5 g/cm$^3$ (at 0.3 psi), measured with the Absorbent Core K(t) Test Method.

13. An absorbent article (200) comprising a topsheet (36), a backsheet (38) and an absorbent core (28) according to any of the preceding claims, and optionally an acquisition layer and/or a distribution layer (54) between the core and topsheet.

14. A method for making an absorbent core (28) according to any of the claims 1-12, the method comprising the steps of:

- providing a high loft central layer (43), a liquid-permeable top layer (41), and a bottom layer (42);
- depositing a first layer of superabsorbent particles on a first side of the high loft central layer;
- laminating the first side of the central layer with one selected from the liquid-permeable top layer (41) and the bottom layer (43);
- optionally depositing a second layer of superabsorbent particles on the second side of the high loft central layer;
- laminating the second side of the central layer with the other of the liquid-permeable top layer (41) or the bottom layer (43) that was not laminated previously;

wherein the superabsorbent polymer particles deposited have a time to reach an uptake of 20 g/g (SAP T20) of less than 220 s, as measured according to the SAP K(t) Test Method as described herein.

15. A method for making an absorbent core according to the preceding claim, wherein the initial density of the central layer is in the range of from 0.05 g/cm$^3$ to 0.15 g/cm$^3$ at 4.14 kPa (0.6 psi) and/or the initial thickness of the central layer at 4.14 kPa (0.6 psi) is of more than 0.30 mm, as measured using Thickness and Density Measurement Method described herein.

**Patentansprüche**

1. Absorptionskern (28) zur Verwendung in einem Absorptionsartikel (20), wobei sich der Absorptionskern in einer Querrichtung (x) und einer Längsrichtung (y) erstreckt und eine Dicke in einer vertikalen Richtung (z) lotrecht zu der Querrichtung und der Längsrichtung aufweist, der Absorptionskern umfassend:

- eine flüssigkeitsdurchlässige obere Schicht (41);
- eine untere Schicht (42);
- eine hochvoluminöse zentrale Schicht (43), die eine Dichte bei einem Druck von 4,14 kPa unter 0,20 g/cm$^3$ zwischen der oberen Schicht und der unteren Schicht aufweist, wie gemäß dem Dicken- und Dichtenmessverfahren, wie hierin beschrieben, gemessen;
- Superabsorberpolymerteilchen zwischen der oberen Schicht und der unteren Schicht, wobei die Superabsorberpolymerteilchen wenigstens teilweise innerhalb der zentralen Schicht (43) verteilt sind;

wobei die Superabsorberpolymerteilchen über eine Zeit von weniger als 220 s verfügen, um eine Aufnahme von 20 g/g (SAP T20) zu erreichen, wie gemäß dem SAP-K(t)-Prüfverfahren, wie hierin beschrieben, gemessen.

2. Absorptionskern nach Anspruch 1, wobei der Absorptionskern über eine Zeit von weniger als 550 s verfügt, um eine Aufnahme von 20 g/g (Kern T20) zu erreichen, wie gemäß dem Absorptionskern-K(t)-Prüfverfahren, wie hierin beschrieben, gemessen.

3. Absorptionskern nach einem der vorstehenden Ansprüche, wobei die zentrale Schicht synthetische Fasern umfasst oder daraus besteht, insbesondere wobei die zentrale Schicht eine kardierte Vliesschicht ist.

4. Absorptionskern nach einem der vorstehenden Ansprüche, wobei die Konzentration der Superabsorberpolymerteilchen in dem Absorptionskern eine bimodale oder multimodale Verteilung in der z-Richtung des Absorptionskerns aufweist, wie durch das hierin offenbarte Mikro-CT-Scanverfahren bestimmt.

5. Absorptionskern nach einem der vorstehenden Ansprüche, wobei die Superabsorberpolymerteilchen aus einem vernetzten Polyacrylatsalz hergestellt sind und vorzugsweise eine Zentrifugenretentionskapazität (CRC) in dem Bereich von 10 g/g bis zu 35 g/g Prüfung aufweisen, wie durch das EDANA-Verfahren NWSP 241.0.R2 (19) gemessen.

6. Absorptionskern nach einem der vorstehenden Ansprüche, wobei die Superabsorberpolymerteilchen eine UPM von wenigstens 10 × 10-7 cm3.s/g, vorzugsweise von wenigstens 15 × 10-7 cm3.s/g aufweisen, wobei die UPM durch die hierin beschriebene Urinpermeabilitätsmessprüfung gemessen wird und/oder wobei die Superabsorberpolymerteilchen ein Absorptionsvermögen gegen Druck von 0,7 psi (AAP@0,7 psi) von mehr als 22 g/g aufweisen, gemäß einer EDANA-Standardprüfung NWSP 242.0 R2 (19) gemessen.

7. Absorptionskern nach einem der vorstehenden Ansprüche, wobei die obere Schicht und/oder die untere Schicht jeweils durch eine Klebstoffschicht (71, 72) an der zentralen Schicht befestigt sind, wahlweise wobei die Klebstoffschicht ebenso wenigstens einen Teil der SAP-Teilchen immobilisiert, die in dem Trockenzustand nicht innerhalb der hochvoluminösen zentralen Schicht verteilt sind.

8. Absorptionskern nach einem der vorstehenden Ansprüche, ferner umfassend eine Hüllenschicht (3), insbesondere eine Vlieshüllenschicht, die wenigstens teilweise die obere, die untere und die zentrale Schicht umhüllt.

9. Absorptionskern nach einem der vorstehenden Ansprüche, wobei der Absorptionskern zu wenigstens 60 Gew.-% die Superabsorberpolymerteilchen, vorzugsweise zu wenigstens 70 Gew.-% oder zu wenigstens 80 Gew.-% die SAP, eines Gesamtgewichts des Kerns, umfasst.

10. Absorptionskern nach einem der vorstehenden Ansprüche, wobei der Absorptionskern Superabsorberpolymerteilchen bei einem Flächengewicht von wenigstens 200 gsm oder wenigstens 300 gsm oder von 300 gsm bis 500 gsm umfasst.

11. Absorptionskern nach einem der vorstehenden Ansprüche, umfassend eine erste zentrale Schicht (431) und eine zweite zentrale Schicht (432) zwischen der oberen Schicht und der unteren Schicht, wobei wenigstens eine der ersten zentralen Schicht und der zweiten zentralen Schicht eine zentrale Schicht nach einem der vorstehenden Ansprüche ist und wobei die erste zentrale Schicht und die zweite zentrale Schicht gleich oder unterschiedlich sein können.

12. Absorptionskern nach einem der vorstehenden Ansprüche, wobei der Absorptionskern eine Kerndichte von weniger als 0,6 g/cm3, vorzugsweise von 0,2 g/cm$^3$ bis weniger als 0,5 g/cm$^3$ (bei 0,3 psi), aufweist, gemessen mit dem Absorptionskern-K(t)-Prüfverfahren.

13. Absorptionsartikel (200), umfassend eine Oberschicht (36), eine Unterschicht (38) und einen Absorptionskern (28) nach einem der vorstehenden Ansprüche und wahlweise eine Aufnahmeschicht und/oder eine Verteilungsschicht (54) zwischen dem Kern und der Oberschicht.

14. Verfahren zum Herstellen eines Absorptionskerns (28) nach einem der Ansprüche 1 bis 12, das Verfahren umfassend die Schritte:

   - Bereitstellen einer hochvoluminösen zentralen Schicht (43), einer flüssigkeitsdurchlässigen oberen Schicht (41) und einer unteren Schicht (42);
   - Abscheiden einer ersten Schicht der Superabsorberpolymerteilchen auf einer ersten Seite der hochvoluminösen zentralen Schicht;
   - Laminieren der ersten Seite der zentralen Schicht mit einer ausgewählt aus der flüssigkeitsdurchlässigen oberen Schicht (41) und der unteren Schicht (43);
   - wahlweise Abscheiden einer zweiten Schicht von Superabsorberpolymerteilchen auf der zweiten Seite der hochvoluminösen zentralen Schicht;
   - Laminieren der zweiten Seite der zentralen Schicht mit der anderen der flüssigkeitsdurchlässigen oberen Schicht (41) oder der unteren Schicht (43), die zuvor nicht laminiert wurde;

   wobei die Superabsorberpolymerteilchen, die abgeschieden werden, über eine Zeit von weniger als 220 s verfügen, um eine Aufnahme von 20 g/g (SAP T20) zu erreichen, wie gemäß dem SAP-K(t)-Prüfverfahren, wie hierin beschrieben, gemessen.

15. Verfahren zum Herstellen eines Absorptionskerns nach dem vorstehenden Anspruch, wobei die anfängliche Dichte der zentralen Schicht in dem Bereich von 0,05 g/cm$^3$ bis 0,15 g/cm$^3$ bei 4,14 kPa (0,6 psi) liegt und/oder die anfängliche Dicke der zentralen Schicht bei 4,14 kPa (0,6 psi) mehr als 0,30 mm beträgt, wie unter Verwendung des hierin beschriebenen Dicken- und Dichtenmessverfahrens gemessen.

## Revendications

1. Âme absorbante (28) pour utilisation dans un article absorbant (20), l'âme absorbante s'étendant dans une direction transversale (x) et une direction longitudinale (y) et ayant une épaisseur dans une direction verticale (z) perpendiculaire à la direction transversale et à la direction longitudinale, l'âme absorbante comprenant :

- une couche supérieure perméable aux liquides (41) ;
- une couche inférieure (42) ;
- une couche centrale (43) à gonflant élevé ayant une masse volumique à une pression de 4,14 kPa inférieure à 0,20 g/cm$^3$ entre la couche supérieure et la couche inférieure, telle que mesurée selon le procédé de mesure d'épaisseur et de masse volumique tel que décrit ici ;
- des particules de polymère superabsorbant entre la couche supérieure et la couche inférieure, dans laquelle les particules de polymère superabsorbant sont au moins partiellement réparties au sein de la couche centrale (43) ;

dans laquelle les particules de polymère superabsorbant ont un temps pour atteindre une absorption de 20 g/g (SAP T20) inférieur à 220 s, tel que mesurée selon le procédé de test de K(t) de PSA tel que décrit ici.

2. Âme absorbante selon la revendication 1, dans laquelle l'âme absorbante a un temps pour atteindre une absorption de 20 g/g (Core T20) inférieur à 550 s, tel que mesurée selon le procédé de test de K(t) d'âme absorbante tel que décrit ici.

3. Âme absorbante selon l'une quelconque des revendications précédentes, dans laquelle la couche centrale comprend ou est constituée de fibres synthétiques, en particulier dans laquelle la couche centrale est une couche de non-tissé cardé.

4. Âme absorbante selon l'une quelconque des revendications précédentes, dans laquelle la concentration en particules de polymère superabsorbant dans l'âme absorbante a une distribution bimodale ou multimodale dans la direction z de l'âme absorbante, telle que déterminée par le procédé de balayage de microtomographie décrit ici.

5. Âme absorbante selon l'une quelconque des revendications précédentes, dans laquelle les particules de superabsorbant sont fabriquées dans un sel de polyacrylate réticulé, et ont de préférence une capacité de rétention centrifuge (CRC) dans la plage allant de 10 g/g jusqu'à 35 g/g, test tel que mesuré par le procédé EDANA NWSP 241.0.R2 (19).

6. Âme absorbante selon l'une quelconque des revendications précédentes, dans laquelle les particules de polymère superabsorbant ont une UPM d'au moins 10 × 10-7 cm3.s/g, de préférence d'au moins 15 × 10-7 cm3.s/g, dans laquelle l'UPM est mesurée par le test de mesure de perméabilité à l'urine décrit ici, et/ou dans laquelle les particules de polymère superabsorbant ont un pouvoir d'absorption sous pression de 0,7 psi (AAP@0,7 psi) de plus de 22 g/g mesuré selon le test normalisé EDANA NWSP 242.0 R2 (19).

7. Âme absorbante selon l'une quelconque des revendications précédentes, dans laquelle la couche supérieure et/ou la couche inférieure sont fixées chacune à la couche centrale par une couche de colle (71, 72), facultativement dans laquelle la couche de colle immobilise également au moins une partie des particules de PSA qui ne sont pas distribuées au sein de la couche centrale à gonflant élevé dans l'état sec.

8. Âme absorbante selon l'une quelconque des revendications précédentes, comprenant en outre une couche d'enveloppement (3), en particulier une couche d'enveloppement non tissée, enveloppant au moins partiellement les couches supérieure, inférieure et centrale.

9. Âme absorbante selon l'une quelconque des revendications précédentes, dans laquelle l'âme absorbante comprend au moins 60 % en poids de particules de polymère superabsorbant, de préférence au moins 70 % ou au moins 80 % en poids de PSA, en poids total de l'âme.

10. Âme absorbante selon l'une quelconque des revendications précédentes, dans laquelle l'âme absorbante comprend des particules de polymère superabsorbant à une masse surfacique d'au moins 200 g/m$^2$, ou d'au moins 300 g/m$^2$ ou de 300 g/m$^2$ à 500 g/m$^2$.

11. Âme absorbante selon l'une quelconque des revendications précédentes, comprenant une première couche centrale (431) et une seconde couche centrale (432) entre la couche supérieure et la couche inférieure, dans laquelle au moins l'une parmi la première couche centrale et la seconde couche centrale est une couche centrale selon l'une quelconque des revendications précédentes, et dans laquelle la première couche centrale et la seconde couche centrale peuvent être identiques ou différentes.

12. Âme absorbante selon l'une quelconque des revendications précédentes, dans laquelle l'âme absorbante a une masse volumique d'âme inférieure à 0,6 g/cm3, de préférence de 0,2 g/cm$^3$ à moins de 0,5 g/cm$^3$ (à 0,3 psi), mesurée avec le procédé de test de K(t) d'âme absorbante.

13. Article absorbant (200) comprenant une feuille de dessus (36), une feuille de fond (38) et une âme absorbante (28) selon l'une quelconque des revendications précédentes, et facultativement une couche de recueil et/ou une couche de répartition (54) entre l'âme et la feuille de dessus.

14. Procédé permettant de fabriquer une âme absorbante (28) selon l'une quelconque des revendications 1 à 12, le procédé comprenant les étapes consistant à :

- fournir une couche centrale (43) à gonflant élevé, une couche supérieure perméable aux liquides (41), et une couche inférieure (42) ;
- déposer une première couche de particules de superabsorbant sur un premier côté de la couche centrale à gonflant élevé ;
- stratifier le premier côté de la couche centrale avec l'une choisie parmi la couche supérieure perméable aux liquides (41) et la couche inférieure (43) ;
- facultativement déposer une seconde couche de particules de superabsorbant sur le second côté de la couche centrale à gonflant élevé ;
- stratifier le second côté de la couche centrale avec l'autre de la couche supérieure perméable aux liquides (41) ou de la couche inférieure (43) qui n'a pas été stratifiée précédemment ;

dans laquelle les particules de polymère superabsorbant déposées ont un temps pour atteindre une absorption de 20 g/g (SAP T20) inférieur à 220 s, tel que mesuré selon le procédé de test de K(t) de PSA tel que décrit ici.

15. Procédé permettant de fabriquer une âme absorbante selon la revendication précédente, dans lequel la masse volumique initiale de la couche centrale est dans la plage allant de 0,05 g/cm$^3$ à 0,15 g/cm$^3$ à 4,14 kPa (0,6 psi) et/ou l'épaisseur initiale de la couche centrale à 4,14 kPa (0,6 psi) est de plus de 0,30 mm, telle que mesurée à l'aide du procédé de mesure d'épaisseur et de masse volumique décrit ici.

**Fig. 1**

**Fig. 2a**

**Fig. 2b**

**Fig. 2c**

**Fig. 3**

Fig. 4

Fig. 5

28

Fig. 6a

28

Fig. 6b

28

Fig. 6c

**Fig. 7a**

**Fig. 7b**

Fig. 8

Fig. 9

**Fig. 10**

Fig. 11

EP 3 881 814 B1

Fig. 12

Fig. 13

1112

1114

1116

1120

1314

1118

1316

1312

1318

1310

Fig. 14

Fig. 15

705

723
708
709
722
701
714
703
720
721
702
719
713
808
717
716
718
710
706
707
704

EP 3 881 814 B1

45

Fig. 16

Fig. 17

Fig. 18

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9511654 A, Tanzer
- WO 2008155699 A1, Hundorf
- US 20150080821 A1, Peri
- WO 2016106021 A1, Bianchi
- WO 2020025401 A, BASF, Ge
- WO 2020032280 A
- WO 2020032281 A
- WO 2020032282 A
- WO 2020032283 A
- WO 2020032284 A, Nippon SHOKUBAI
- EP 2901992 A1, Ontex
- WO 200071067 A, KIM DOO-HONG
- US 7744576 B
- US 20110268932 A1
- US 20110319848 A1
- US 20110250413 A1
- WO 2006083584 A
- WO 2007047598 A
- WO 2007046052 A
- WO 2009155265 A
- WO 2009155264 A
- EP 3391961 A1, Kamphus, P&G
- US 5300565 A
- US 5180622 A, Berg
- US 5149334 A
- US 5102597 A, Roe
- US 5492962 A, Lahrman
- EP 3056521 B1, Kim
- EP 1512712 B1, Koji
- US 10414876 B2, Jang
- US 7429009 B2, Nagasawa
- EP 220224911 A, Higashimoto
- EP 2011803 B1, Handa
- EP 2944376 A1
- US 4340706 A
- US 5849816 A
- US 20090192035 A
- US 20090258994 A
- US 20100068520 A
- WO 2015041784 A1
- CN 101797201
- US 8585666 B2, Weisman
- EP 2535698 A1, Ehrnsperger
- EP 2535698 A

**Non-patent literature cited in the description**

- Absorbency. Elsevier, 1982, 42-43
- **J.M. COULSON** ; **J.F. RICHARDSON**. Chemical Engineering. Pergamon Press, 1978, vol. II, 122-127
- *CHEMICAL ABSTRACTS*, 7647-14-5
- *CHEMICAL ABSTRACTS*, 68439-50-9